(19) Europäisches Patentamt
European Patent Office
Office européen des brevets

(11) Numéro de publication : **0 558 411 A1**

# (12) DEMANDE DE BREVET EUROPEEN

(21) Numéro de dépôt : **93400478.9**

(51) Int. Cl.$^5$ : **C12N 9/88**, C12P 19/26, C08B 37/00

(22) Date de dépôt : **24.02.93**

(30) Priorité : **26.02.92 FR 9202255**

(43) Date de publication de la demande : **01.09.93 Bulletin 93/35**

(84) Etats contractants désignés : **AT BE CH DE DK ES FR GB GR IE IT LI LU MC NL PT SE**

(71) Demandeur : **ELF SANOFI**
**32-34, rue Marbeuf**
**F-75008 Paris (FR)**

(72) Inventeur : **Salome, Marc Louis Victor**
**Les Ormes II Bât. F1**
**F-31320 Castanet Tolosan (FR)**
Inventeur : **Lelong, Philippe**
**Les Ormes - Bât. A6**
**F-31320 Castanet Tolosan (FR)**

(74) Mandataire : **Le Guen, Gérard et al**
**CABINET LAVOIX 2, place d'Estienne d'Orves**
**F-75441 Paris Cédex 09 (FR)**

## (54) Enzyme destinée à la fragmentation du N-acétylhéparosane, obtention des préparations contenant cette enzyme et procédés de fragmentation utilisant cette enzyme.

(57) L'invention concerne une enzyme capable de fragmenter un N-acétylhéparosane de haute masse moléculaire.
Cette enzyme a été obtenue par la souche Escherichia coli (K5), souche SEBR 3282.

EP 0 558 411 A1

La présente invention concerne une enzyme destinée à la fragmentation du N-acétylhéparosane, l'obtention des préparations contenant cette enzyme ainsi que des procédés de fragmentation utilisant cette enzyme.

Il est connu que certaines bactéries de l'espèce Escherichia coli, produisent un polysaccharide capsulaire, habituellement appelé K5, qui est une famille de polymères constituée d'unités répétées β-D-glucuronyl-1,4-α-N-acétyl-D-glucosaminyl (1,4), (W.F. Vann et al, Eur. J. Biochem, 1981, 116, 359-364), de structure (a) :

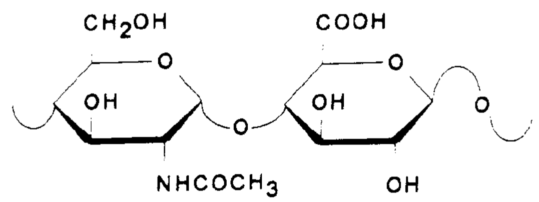
(a)

Ce polysaccharide sera appelé ici "N-acétylhéparosane". Ce produit possède une masse moléculaire comprise entre $10^5$ et $2. 10^6$ Da, et au niveau des unités "acide uronique", une structure très régulière composée uniquement d'acide D-glucuronique (W.F. Vann et al, Eur. J. Biochem, 1981, 116, 359-364, et la demande de brevet EP-A-0 333 243).

Le N-acétylhéparosane est surtout utile comme matière première pour l'industrie pharmaceutique mais, pour cette utilisation, il a une masse moléculaire trop élevée.

Il est connu que le N-acétylhéparosane (polysaccharide K5) peut être fragmenté par une lyase phagique provenant d'un phage spécifique de la souche Escherichia coli (K5), mais cette fragmentation est très poussée et conduit à une disparition importante des fragments de masse moléculaire de 5000 Da vers des chaînes beaucoup plus petites (D. Gupta et al, FEMS Miuobiology Letters, 1983, 16, 13-17. Cette fragmentation est utilisée dans la demande de brevet EP-A-0 333 243 pour la préparation de fragments contenant au maximum 10 unités saccharidiques.

On connaît aussi que des oligosaccharides possédant à l'extrémité non réductrice une unité glucuronique ayant une double liaison entre les carbones 4 et 5, peuvent être obtenus par dépolymérisation enzymatique des polysaccharides, en utilisant comme enzyme, l'enzyme obtenue à partir du Bacillus circulans et notamment à partir de la souche Bacillus circulans NCIB 12482. Cette enzyme agit sur les liaisons glucose-acide glucuronique (EP-0 294 879). Une enzyme obtenue à partir d'une culture de Bacillus polymyxa est aussi décrite dans le brevet US-3.812.012.

De manière surprenante il a été constaté que les cultures d'Escherichia coli (K5), souche SEBR 3282, produisent dans certaines conditions environnementales, une enzyme qui fragmente le N-acétylhéparosane au cours de la culture en fermenteur. De manière non moins surprenante, il a aussi été constaté que les fragments de N-acétylhéparosane se situent autour d'un pic de masse moléculaire d'environ 5000 Da, rassemblant à quelques unités disaccharidiques près, environ 70 % du produit.

Enfin, de manière toujours surprenante, il a été constaté qu'une préparation de cette enzyme convenablement solubilisée, permettait d'obtenir des fragments de masse moléculaire plus élevée par rapport aux fragments obtenus spontanément, c'est à dire avec l'enzyme non solubilisée. En effet, des préparations d'enzymes solubilisées permettent de moduler la fragmentation et d'obtenir des fragments dont la majorité a une masse moléculaire supérieure d'au moins 1000 à 3000 Da par rapport à la masse moléculaire des fragments obtenus spontanément.

Ainsi, la présente invention a pour objet une enzyme qui permet la fragmentation modulée d'un N-acétylhéparosane de haute masse moléculaire et l'obtention de fragments de N-acétylhéparosane de masse moléculaire désirée, avec un très bon rendement. L'enzyme de la présente invention est donc utile pour préparer des N-acétylhéparosanes de petite masse moléculaire à partir d'un N-acétylhéparosane de haute masse moléculaire, pour les raisons suivantes:

- le N-acétylhéparosane de haute masse moléculaire s'obtient sur milieu synthétique peu coûteux avec des rendements supérieurs à ceux d'un milieu complexe, milieu nécessaire à l'obtention d'un N-acétylhéparosane de petite masse moléculaire;
- le N-acétylhéparosane de haute masse moléculaire est techniquement plus facile à manipuler que celui de petite masse moléculaire;
- la fragmentation in vitro sépare la phase de production de celle de fragmentation, ce qui permet de maîtriser et d'optimiser les deux phases tout en offrant une grande latitude sur les caractéristiques du produit recherché, par exemple, sur sa masse moléculaire ;
- l'enzyme et le N-acétylhéparosane sont particulièrement stables ce qui autorise des recyclages multiples, éventuellement nécessaires dans la mise en oeuvre de procédés dynamiques, au cours desquels,

les processus de fragmentation et de fractionnement sont mis en oeuvre simultanément ou successivement tout en s'affranchissant des contraintes de temps liées à l'emploi d'un bioréacteur.

Plus précisément l'enzyme de la présente invention est caractérisée en ce que:

- elle est obtenue à partir d'une souche Escherichia coli (K5), la souche Escherichia coli SEBR 3282, ou d'un mutant spontané ou induit de cette souche.
- sa masse moléculaire est comprise entre 62000 et 70000 Da, plus précisément entre 65000 Da et 66000 Da (± 1500 Da),
- son point isoélectrique se situe dans la zone de pH comprise entre 4,7 et 5,4 unités pH, plus précisément, 5,1 unités pH,
- elle est une éliminase, plus précisément une endo-β-éliminase.

L'enzyme de la présente invention est caractérisée aussi en ce qu'elle agit de la façon suivante:

- elle est d'origine membranaire,
- la température de son fonctionnement optimal (activité maximale) est voisine de 37°C et la température à laquelle elle est inactivée est d'environ 60°C,
- la zone optimale de pH pour son fonctionnement se situe entre les valeurs pH 6 et pH 7, et plus précisément entre pH 6,6 et pH 6,8,
- pour son fonctionnement, la zone optimale de concentration en ions monovalents ou divalents se situe au voisinage de 0,2 M, et plus précisément 0,15 M pour les ions divalents et 0,25 M pour les ions monovalents.

De plus, l'enzyme de l'invention est caractérisée en ce que:

- elle ne permet pas de scinder le N-acétylhéparosane en dessous d'une certaine taille, taille qui correspond à une masse moléculaire d'environ 1000 à 1500 Da,
- elle est capable d'agir sur le N-acétylhéparosane de haute masse moléculaire et de le fragmenter en l'absence de corps bactériens in vitro.

L'enzyme, objet de la présente invention, est une enzyme obtenue d'une culture d'Escherichia coli, notamment d'Escherichia coli SEBR 3282. Cette souche est une souche dérivée de la souche Bi 8337-41 (O10:K5:H4) ATCC 23506 (décrite par D.S. Gupta et al FEMS Microbiology Letters, 1982, 14, 75-78 et W. Vann Eur. J. Biochem., 1981, 116, 359-364).

La souche Escherichia coli (K5) SEBR 3282 répond positivement au test de typage par le phage K5 spécifique selon la méthode de B. Kaiser et al (J. Clin. Microbiol., (1984), 19, 2, 264-266). Il s'agit donc bien d'une souche Escherichia coli (K5). Cette souche a été déposée auprès de la CNCM de l'Institut Pasteur, Paris, France, sous le N° I-1013. Il est aussi possible d'isoler cette enzyme d'un mutant, soit spontané, soit induit de la souche Escherichia coli SEBR 3282, ou encore par d'autres souches d'Escherichia coli (K5) appropriées et notamment par la souche Bi 8337-41 (010:K5:H4) ATCC 23506. Il est aussi envisageable d'obtenir cette enzyme par la souche Bi 626-42 (012:K5:NM) ATCC 23508.

La masse moléculaire de cette enzyme, évaluée par chromatographie d'exclusion, est comprise entre 62000 et 70000 Da, et elle est plus précisément d'environ 65000 Da à 66000 Da (± 1500 Da).

Le point isoélectrique de l'enzyme, objet de la présente invention, se situe à un pH de 4,7-5,4 et plus précisément, 5,1 unités pH.

L'enzyme de l'invention permet de fragmenter le N-acétylhéparosane et d'obtenir des fragments comportant à l'extrémité non réductrice un reste d'acide glucuronique ayant une double liaison entre les carbones 4 et 5 (élimination du groupe OH). De telles enzymes ne font pas intervenir l'eau dans la réaction chimique considérée et sont dites de type éliminase. L'enzyme, objet de la présente invention est donc une éliminase et notamment une endo-β-éliminase.

Le préfixe "endo" est utilisé pour indiquer que l'enzyme, objet de la présente invention est capable de fragmenter le N-acétylhéparosane à une distance considérable de l'extrémité de la molécule. Plus particulièrement, l'enzyme de la présente invention ne permet pas de scinder le N-acétylhéparosane en dessous d'une certaine taille, qui correspond à une masse moléculaire d'environ 1000 Da à 1500 Da. Ainsi, l'enzyme, objet de la présente invention, lors d'une fragmentation totale d'un N-acétylhéparosane de haute masse moléculaire, permet d'obtenir des fragments de N-acétylhéparosane de masses moléculaires comprises entre 1000 Da et 10000 Da et plus spécialement d'obtenir des fragments ayant des masses moléculaires regroupées à 70 % aux environs de 5000 Da.

Le N-acétylhéparosane de haute masse moléculaire qui peut être fragmenté par l'enzyme objet de la présente invention, peut être obtenu comme décrit par W. F. Vann et al, Eur. J. Biochem, 1981, 116, 359-364, et dans la demande de brevet EP-A-0 333 243, ou bien par fermentation de la même souche Escherichia coli (K5) SEBR 3282 utilisée pour la préparation de l'enzyme de la présente invention.

Par action de l'enzyme de la présente invention, le N-acétylhéparosane de haute masse moléculaire est fragmenté en des fragments ayant une masse moléculaire majoritaire d'environ 5000 Da. Par le terme "masse

moléculaire majoritaire" on entend la masse moléculaire qui correspond au maximum (pic) du profil chromatographique obtenu lors de la détermination de la masse moléculaire du N-acétylhéparosane par chromatographie en perméation de gel (CPG). Les fragments ainsi obtenus présentent à l'extrémité non réductrice une unité glucuronique ayant une double liaison entre les carbones 4 et 5.

Cette enzyme permet en particulier d'obtenir en fin de culture, à partir d'un N-acétylhéparosane de haute masse moléculaire, des fragments ayant dans leur majorité, des tailles identiques, qui correspondent à une masse moléculaire d'environ 5000 Da. Donc, l'enzyme de l'invention doit être capable d'intervenir au niveau de la macromolécule de N-acétylhéparosane à une distance donnée de l'extrémité de cette macromolécule. Il s'agit donc d'une endo-éliminase qui permet d'obtenir une majorité de fragments constitués par un nombre de motifs β-D-glucuronyl -1,4-α-N-acétyl-D-glucosaminyl (1,4) identique.

Le suivi de la fragmentation spontanée du N-acétylhéparosane au cours d'une culture en milieu complexe fait également apparaître un phénomène de lyse légère qui permet difficilement de prévoir où l'enzyme concernée est localisée dans la cellule : cytoplasme, périplasme, membrane ou encore milieu de culture extracellulaire.

Les méthodes employées pour localiser l'endo-β-éliminase sont définies et regroupées dans le Schéma 1.

## SCHEMA 1

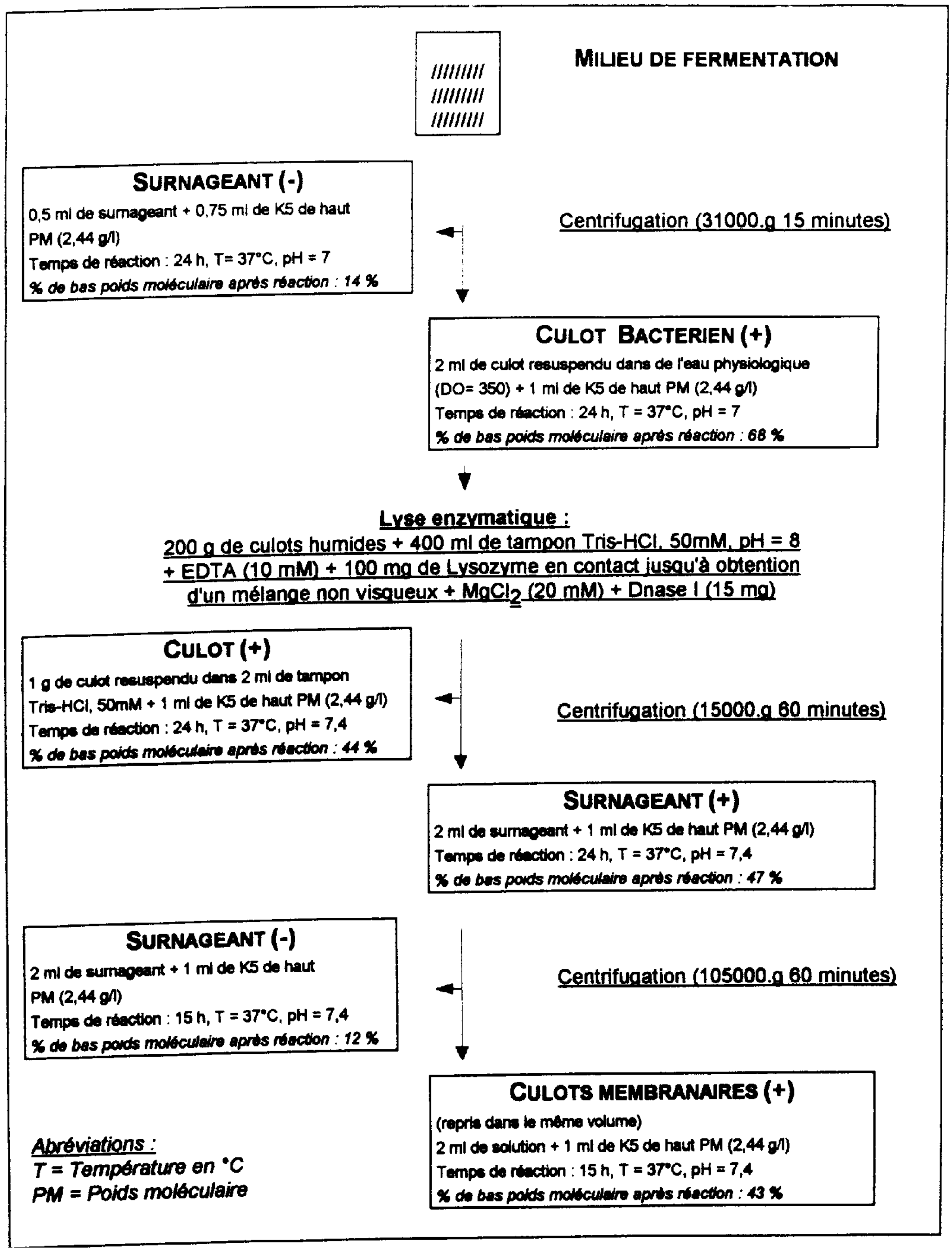


On observe que:

- le surnageant des suspensions de culture de la souche Escherichia coli (K5) SEBR 3282 sur "milieu complexe" contient de faible quantité d'endo-β-éliminase;

- l'essentiel de l'activité enzymatique se situe dans les culots bactériens bruts ;
- les préparations de membranes lipidiques obtenues à partir des surnageants de lyse des culots lavés, renferment l'essentiel de l'activité de type β-éliminase;

Par conséquent:

- à l'origine l'enzyme est membranaire et sans autre manipulation, l'essentiel de l'activité se retrouve dans les membranes lipidiques des bactéries;
- au cours de cultures en milieu liquide, et particulièrement sous agitation, ainsi que dans des conditions de lyse des corps bactériens, une partie de l'activité peut se retrouver et s'exercer en dehors des corps bactériens ;
- il n'est pas nécessaire de concentrer l'enzyme, pour l'obtenir il suffit d'isoler les membranes lipidiques.

L'enzyme, objet de la présente invention, est donc une enzyme d'origine membranaire, l'essentiel de l'activité se retrouvant dans les membranes lipidiques des bactéries. Toutefois, au cours de cultures en milieu liquide, et particulièrement sous agitation, ainsi que dans des conditions de lyse des corps bactériens, une partie de l'activité peut se retrouver et s'exercer en dehors des corps bactériens.

La solubilisation de l'enzyme de la présente invention a été établie à l'aide de différents détergents selon les techniques connues (Biotechnology and Applied Biochemistry, 1990, 12, 599-620), à partir d'un culot membranaire.

Ainsi par exemple, les préparations d'enzymes solubilisées par les détergents tels que le Triton X-100®, le Triton X-114®, DOC, NP-40®, Tween 80®, tous à 2 % et le chlorhydrate de guanidine 0,1 M sont actives. On peut aussi obtenir l'enzyme sous forme solubilisée, en soumettant le culot bactérien brut à une lyse alcaline.

La détermination du point isoélectrique (pHi) de l'enzyme a été effectuée selon la méthode de "chromatofocusing" dans une zone de pH comprise entre 3,5 et 9 Le pHi de l'enzyme ainsi déterminé se situe entre pH 4,7 et pH 5,4, et plus précisément à 5,1 unités pH.

La masse moléculaire de l'enzyme de la présente invention a été déterminée par chromatographie, en perméation de gel (CPG) et se situe entre 62000 et 70000, le pic des protéines étant à environ 65000-66000 Da (± 1500 Da).

L'enzyme de la présente invention, sous forme de préparation membranaire a été également soumise aux tests pour étudier les facteurs environnementaux exerçant une influence sur son activité. Aussi, on connaît l'influence de la température, du pH, des ions monovalents, notamment de l'ion $Na^+$, des ions divalents notamment de l'ion $Ca^{2+}$. On a aussi déterminé la constante de Michaelis, et on a observé la stabilité de l'enzyme dans certaines conditions.

La température optimale de fonctionnement de l'enzyme (activité maximale) objet de la présente invention, est voisine de 37°C. La température d'inactivation de l'enzyme est de 60°C, et à la température de 20°C l'enzyme conserve 40 % de son activité versus celle à 37°C.

La zone optimale de pH de fonctionnement de l'enzyme, objet de la présente invention, se situe entre les valeurs pH 6 et pH 7, et plus précisément pH 6,6 à pH 6,8.

Par ailleurs, la zone optimale de concentration en ions monovalents et plus particulièrement en ions sodium, pour le fonctionnement de l'enzyme objet de la présente invention, se situe au voisinage de 0,2 M, et plus précisément à environ 0,25 M.

Pour les ions divalents, la zone optimale de concentration pour le fonctionnement d'enzyme, se situe aussi au voisinage de 0,2 M et plus précisément à environ 0,15 M. En effet, lorsque l'on utilise l'enzyme mise en solution dans du chlorure de calcium à 0,2 M, on observe une activation d'environ 100 % par rapport au témoin sans ion $Ca^{2+}$.

L'invention concerne aussi un procédé d'obtention de l'enzyme, objet de la présente invention, et notamment l'obtention de préparations contenant cette enzyme à partir d'une culture d'Escherichia coli (K5), souche SEBR 3282 ou à partir d'un mutant soit spontané soit induit de la souche SEBR 3282. Toutefois, il est aussi possible d'obtenir cette enzyme par d'autres souches d'Escherichia coli (K5) appropriées, par exemple par la souche Bi 8337-41 (010:K5:H4) ATCC 23506. Il est aussi envisageable d'obtenir cette enzyme par la souche Escherichia coli (K5) Bi 626-42 (012: K5: NM) ATCC 23508.

Plus particulièrement, l'invention concerne aussi un procédé d'obtention de l'enzyme, objet de la présente invention, sous forme de culots bactériens bruts la contenant, caractérisé en ce que l'on effectue une culture d'Escherichia coli (K5) SEBR 3282 ou d'un mutant spontané ou induit de cette souche ou d'une autre souche d'Escherichia coli (K5) appropriée, dans un milieu de culture favorable à la formation de cette enzyme, et que l'on isole le culot de la culture par centrifugation, par exemple à 10000.g.

L'invention concerne aussi une préparation contenant l'enzyme objet de la présente invention, constituée d'un culot bactérien brut obtenu d'une culture d'Escherichia coli (K5) SEBR 3282 ou d'un mutant spontané ou induit de cette souche, contenant cette enzyme.

L'invention concerne aussi un procédé d'obtention de l'enzyme objet de la présente invention, sous forme

de lysats bruts la contenant, caractérisé en ce que l'on effectue une culture d'Escherichia coli (K5) SEBR 3282 ou d'un mutant spontané ou induit de cette souche, dans un milieu de culture favorable à la formation de cette enzyme, on isole le culot de la culture et on soumet ce culot à une lyse.

Les préparations contenant l'enzyme, objet de la présente invention, constituées d'un lysat brut obtenu par lyse d'un culot bactérien brut contenant cette enzyme, font aussi partie de la présente invention.

L'invention concerne aussi une préparation contenant l'enzyme, objet de la présente invention, constituée d'une préparation membranaire obtenue par lyse d'un culot bactérien contenant cette enzyme et, ensuite par séparation des membranes du lysat du culot bactérien.

L'invention concerne également les préparations contenant l'enzyme sous forme solubilisée.

Ces préparations peuvent être obtenues en solubilisant l'enzyme contenue dans une préparation membranaire à l'aide de détergents anioniques ou cationiques.

Elles peuvent être aussi obtenues en soumettant un culot bactérien brut contenant cette enzyme, à une lyse partielle en milieu alcalin.

Le terme "préparation contenant l'enzyme" inclut bien les préparations de l'enzyme sous forme de culots bactériens bruts comme aussi les lysats bruts obtenus à partir d'une culture d'Escherichia coli (K5), souche SEBR 3282, ou par un mutant soit spontané soit induit de la souche.

Le terme "préparation contenant l'enzyme" inclut également les préparations membranaires obtenues à partir d'un lysat brut contenant l'enzyme objet de la présente invention.

Le terme "préparation contenant l'enzyme" inclut également les préparations de l'enzyme solubilisée obtenues par des préparations membranaires contenant l'enzyme objet de la présente invention, comme aussi les préparations de l'enzyme solubilisée, obtenues après lyse alcaline des culots bactériens bruts contenant l'enzyme objet de la présente invention.

Le terme "préparation contenant l'enzyme" inclut aussi toute autre préparation contenant l'enzyme seule ou en association avec une autre enzyme ou des substances organiques ou minérales.

Pour obtenir un culot bactérien brut contenant l'enzyme, objet de la présente invention, on utilise le culot de la suspension d'une culture d'Escherichia coli (K5), souche SEBR 3282 effectuée dans des conditions environnementales favorables à la formation de l'enzyme objet de la présente invention. Ce culot est isolé de la suspension d'une culture d'Escherichia coli (K5) souche SEBR 3282 par des méthodes classiques, par exemple par centrifugation.

En effet, une activité de type éliminase ne s'observe, au cours des cultures d'Escherichia coli (K5), souche SEBR 3282, que sous certaines conditions:

1 - le milieu de culture doit contenir un inducteur de la synthèse de l'enzyme qui peut être, soit complexe comme l'extrait de levure, soit pur comme la N-acétylglucosamine;

2- le milieu de culture doit en outre contenir un mélange d'acides aminés comme par exemple, l'hydrolysat de caséine (HY-CASE SF - Sheffield® USA), ou encore un mélange d'acides aminés synthétiques à partir d'acides aminés purs comme décrits dans la formulation du milieu E;

3- la source carbonée utilisée peut être le glucose ou le glycérol. Ce dernier composé ne permet cependant pas d'obtenir une activité d'éliminase notable.

Pour observer l'activité maximale de l'éliminase, plusieurs éléments doivent donc intervenir en synergie:

- les éléments inducteurs proprement dits;
- les éléments régulant la synthèse, tels que les sources azotées et carbonées;
- les facteurs environnementaux influençant l'activité de l'enzyme elle même ou de son interaction avec le substrat, tels que les ions monovalents ou divalents, la température, le pH ou autres.

Par ailleurs, la cinétique de l'activité éliminase au cours de la culture montre que, soit la synthèse de l'enzyme a lieu principalement pendant la phase post exponentielle de la culture, soit l'enzyme persistante est activée pendant cette phase de la culture.

Il apparaît donc que l'induction de l'enzyme peut être recherchée de manière rationnelle, à l'aide de milieux synthétiques qui remplissent simultanément les conditions décrites ci-dessus

Le milieu E, dont la composition est donnée dans le Tableau I ci-après, permet d'envisager la formulation de milieux de culture performants, exempts de matières premières complexes d'origine animale.

## TABLEAU I

**Composition et préparation du milieu E**

**MILIEU E**

Le milieu E est obtenu par la réunion des deux solutions stériles 1 et 2 suivantes :

Solution N°1

Dans 700 ml d'eau ultra-purifiée, dissoudre dans l'ordre :

| | | |
|---|---|---|
| Complexant : N'[Tris-(hydroxyméthyl) méthyl] glycine (Tricine commercialisé par Fluka®) | 360 | mg |
| $K_2HPO_4$ | 790 | mg |
| $MgCl_2$, $6H_2O$ | 620 | mg |
| $K_2SO_4$ | 610 | mg |
| $FeSO_4$, $7H_2O$ | 25 | mg |
| $CaCl_2$, $2H_2O$ | 2 | mg |
| NaCl | 500 | mg |
| KCl | 5000 | mg |
| KI | 10 | mg |
| Yeast extract | 18000 | mg |

Chauffer légèrement, ajuster le pH à 7,4 puis compléter le volume à 750 ml avec de l'eau ultra-purifiée, et autoclaver 30 minutes à 120°C.

Solution N°2

dissoudre dans 100 ml :

| | | |
|---|---|---|
| Acide glutamique | 17,8 | g |
| Proline | 1,6 | g |
| Méthionine | 0,4 | g |
| Glycine | 0,75 | g |
| Arginine | 1,25 | g |
| Cystéine | 0,20 | g |
| $K_2HPO_4$ | 1,56 | g |
| Solution d'oligo-éléments (Cf. Tableau II ci-après) | 1 | ml |
| Glucose | 18 | g |

Ajuster le pH à 7,4 avec KOH. Compléter le volume à 167 ml avec de l'eau ultra-purifiée, et effectuer une filtration stérilisante sur membrane de 0,2 µm.

## TABLEAU II

| Préparation de la solution d'oligo-éléments | | |
|---|---|---|
| dans 800 ml d'eau ultra-purifiée dissoudre (dans l'ordre) : | | |
| $H_3BO_3$ | 500 | mg |
| $Na_2MoO_4,2H_2O$ | 1930 | mg |
| $CoCl_2,6H_2O$ | 11850 | mg |
| $CuSO_4,5H_2O$ | 25 | mg |
| $ZnSO_4,7H_2O$ | 2000 | mg |
| $AlCl_3,6H_2O$ | 2410 | mg |
| Ajouter 100 ml d'acide chlorhydrique de densité 1,19 et compléter à 1000 ml avec de l'eau ultra-purifiée. | | |

Comme milieu de culture on peut aussi citer, à titre d'exemple non exclusif, un milieu complexe tel qu'il est décrit dans la présente invention et notamment le "milieu D" pour la préculture de la souche Escherichia coli (K5), SEBR 3282 et le "milieu C" pour la culture de cette préculture.

Pour obtenir un lysat brut contenant l'enzyme objet de la présente invention, on utilise le culot de la suspension d'une culture d'Escherichia coli (K5), souche SEBR 3282, effectuée dans des conditions environnementales favorables à la formation de l'enzyme objet de la présente invention, et plus particulièrement les culots bactériens bruts contenant ladite enzyme. En effet, à la fin de cette culture, le culot de la suspension de la culture de la souche SEBR 3282 est récupéré par centrifugation puis lysé, par exemple par addition de lysozyme. Ce lysat brut ainsi obtenu peut être directement utilisé.

Les préparations membranaires contenant l'enzyme objet de la présente invention, sont obtenues à partir du lysat brut, par centrifugations successives permettant d'isoler les culots membranaires qui sont ensuite solubilisés, soit dans l'eau ultra-purifée, soit dans des tampons appropriés tel que par exemple le tampon tris-HCl pH 7,2. Il est également possible d'utiliser des solutions aqueuses contenant des ions monovalents ou divalents notamment de ions $Na^+$ ou $Ca^{2+}$.

Les préparations d'enzymes solubilisées sont obtenues à partir de préparations membranaires contenant cette enzyme. En effet, l'enzyme objet de la présente invention, peut-être solubilisée à partir de préparations membranaires et obtenues sous forme active.

Pour la solubiliser, on peut utiliser des détergents, des sels, des enzymes ou tout autre moyen connu de l'homme de l'art. En effet, l'enzyme, objet de la présente invention, peut-être solubilisée à partir de préparations lipidiques en utilisant certains détergents sans être dénaturée.

Comme détergents on peut plus spécialement utiliser les détergents non ioniques A titre d'exemples on peut citer le Triton X-100®, le Triton X-114®, le Déoxycholate (DOC), le NP-40®, le Tween 80® et la Guanidine-HCl 0,1 M. Toutefois la mise en évidence de l'activité enzymatique de l'enzyme objet de la présente invention, nécessite l'élimination des résidus de détergents en utilisant par exemple des colonnes affines spécifiques du commerce.

L'enzyme faisant l'objet de la présente invention, peut aussi être solubilisée de manière non dénaturée sans faire appel aux détergents, par lyse partielle en milieu alcalin. Comme milieu alcalin (pH environ 11), on peut utiliser des solutions de bases minérales fortes, telles que l'hydroxyde de sodium ou l'hydroxyde de potassium. On préfère l'hydroxyde de potassium.

L'invention concerne aussi des procédés de fragmentation des N-acétylhéparosanes de haute masse moléculaire mettant en oeuvre des préparations de l'enzyme objet de la présente invention.

Ces procédés peuvent mettre en oeuvre l'enzyme objet de la présente invention soit dans des milieux de culture in vivo lors de la production de N-acétylhéparosanes, soit in vitro dans le cas où la phase de production de N-acétylhéparosane est séparée de celle de fragmentation. Dans ce dernier cas, les préparations de l'enzyme objet de la présente invention, ne sont utilisées qu'à la phase de fragmentation.

La réaction enzymatique est effectuée entre pH 4,0 et pH 9,0, de préférence entre pH 6,4 et pH 7,4.

La mise en évidence in vivo de l'enzyme a été effectuée par la fragmentation provoquée d'une quantité de N-acétylhéparosane de haute masse moléculaire ajoutée à une suspension de culture présentant une activité éliminasique.

Il a été confirmé que l'enzyme ainsi obtenue coupe le N-acétylhéparosane de haute masse moléculaire de façon à obtenir un N-acétylhéparosane composé majoritairement de fragments de masse moléculaire de 5000 Da. Il ne s'agit donc pas d'un arrêt de la synthèse du N-acétylhéparosane de haute masse moléculaire par la bactérie, mais d'une modification ultérieure du polymère.

On a trouvé, de façon surprenante, que la masse moléculaire du N-acétylhéparosane obtenu par fragmentation opérée par l'enzyme de la présente invention peut être modulée en utilisant une préparation de l'enzyme solubilisée ou bien en réalisant la réaction enzymatique en présence de cations, notamment des ions $Na^+$.

Ainsi, selon un aspect ultérieur, la présente invention concerne un procédé pour la préparation de N-acétylhéparosane ayant des masses moléculaires regroupées, se situant notamment entre 2000 et 10000 Da, caractérisé en ce que l'on traite le N-acétylhéparosane de haute masse moléculaire (polysaccharide K5) avec l'enzyme de la présente invention en présence d'une solution de chlorure de sodium ayant notamment une molarité de 0,2 à 0,5 M.

Le terme "masses moléculaires regroupées entre 2000 et 10000 Da", tel qu'utilisé ici, se rapporte aux différents N-acétylhéparosanes ayant une masse moléculaire avec une courbe de distribution assez étroite, par exemple 1000 à 3000 Da avec masse moléculaire majoritaire à 2000 Da, de 3200 à 4000 avec masse moléculaire majoritaire à 3600 Da, de 3900 à 4700 Da avec masse moléculaire majoritaire à 4300 Da, de 6000 à 8000 Da avec masse moléculaire majoritaire à 7000 Da, de 8000 à 10000 Da avec masse moléculaire majoritaire à 9200 Da. Il est entendu que ces masses moléculaires se réfèrent à la majorité des constituants, à savoir entre environ 50 % et environ 90 %. La masse moléculaire des produits ainsi obtenus dépend en particulier de la molarité du milieu en chlorure de sodium du milieu réactionnel comme aussi de la forme solubilisée ou non de l'enzyme.

Le procédé de la présente invention peut aussi être conduit en fixant le N-acétylhéparosane de haute masse moléculaire sur une colonne de résine échangeuse d'ions et en traitant le produit fixé sur la colonne avec une solution de l'enzyme de la présente invention.

En éluant avec une solution contenant du chlorure de sodium de 0,2 à 0,5 M, on obtient le N-acétylhéparosane ayant la masse moléculaire souhaitée, dépendant de la molarité de la solution en chlorure de sodium utilisée. Pour cette variante, on peut utiliser comme résine, des résines échangeuses d'anions, par exemple une résine type Q-Sepharose® ou d'autres résines équivalentes.

Par ailleurs, compte tenu du fait que l'enzyme, objet de la présente invention est active dans une large gamme de concentrations en chlorure de sodium, on peut envisager l'utilisation de l'enzyme dans des procédés qui permettent une fragmentation et un fractionnement simultané du N-acétylhéparosane fixé sur la colonne.

L'invention concerne aussi des procédés de préparation de N-acétylhéparosanes de petites masses moléculaires utilisant l'enzyme objet de la présente invention, sous forme immobilisée.

L'invention concerne également l'utilisation de l'enzyme pour la fragmentation de N-acétylhéparosanes de haute masse moléculaire.

Pour la préparation des N-acétylhéparosanes de petites masses moléculaires, on peut aussi envisager un procédé associant une étape de fragmentation d'un N-acétylhéparosane de haute masse moléculaire par l'enzyme, objet de la présente invention et une étape de fractionnement alcoolique ou une étape d'ultrafiltration.

Les exemples suivants illustrent l'invention sans toutefois la limiter. Dans la PREPARATION, et dans les EXEMPLES, on a utilisé des dénominations commerciales qui sont explicitées ci-après:

| | |
|---|---|
| Triton X-100® | : polyéthylène glycol tert-octylphényl éther (Rohm & Hass Co.) |
| Triton X-114® | : polyéthylène glycol tert-octylphényl éther (Rohm & Hass Co.) |
| DOC | : sel de sodium d'acide déoxycholique |
| NP-40® | : Nonidet® P-40, éthylphénylpolyéthylèneglycole (Shell) |
| TRIS-HCl | : Tris(hydroxyméthyl)aminométhane hydrochloride |
| Tween 80® | : polyoxyéthylène sorbitanmonoolate (Atlas Chem. Ind. Inc) |
| Struktol J 673® | : antimousse de (Schill & Seilacher) - Allemagne/Hambourg |

## PREPARATION

### N-acétylhéparosane majoritairement de haute masse moléculaire

On ensemence 400 ml du milieu B, de composition précisée dans le Tableau III ci-après, avec la souche Escherichia coli (K5) SEBR 3282 déposée auprès de la CNCM de l'Institut Pasteur, Paris - France, sous le N° I-1013, et on incube sous agitation pendant 2 h à 37°C. La préculture obtenue est alors transférée dans un fermenteur de 18,5 l contenant 11 l du milieu A, de composition précisée aussi dans le Tableau III ci-après, et on incube pendant 6 h 30 minutes à 37°C et pH égal à 7,2, la pression partielle en oxygène étant maintenue

à 40 mmHg par régulation de l'injection d'air (jusqu'à 20 l/minutes) et de l'agitation. On ajoute alors du glycérol en introduisant de façon continue une solution stérile contenant 500 g/l de glycérol à raison de 18 g/h pendant 16-17 heures.

On poursuit la culture dans les mêmes conditions de température, de pH et de pression partielle en oxygène jusqu'à consommation quasi totale du glycérol. Le suivi de la densité optique (DO) à λ = 600 nm de la suspension de culture après la fin de l'ajout en glycérol, montre un état stationnaire ou de lyse légère jusqu'à l'arrêt de la culture à 28-30 h d'âge en fermenteur.

## TABLEAU III

**Composition et préparation du milieu A et du milieu B.**

**MILIEU A**

Dans 900 ml d'eau ultra-purifiée dissoudre dans l'ordre :

| | | |
|---|---|---|
| NTA (acide nitrilotriacétique)* | 1000 | mg |
| $K_2HPO_4$ | 790 | mg |
| Acide glutamique | 11000 | mg |
| $MgCl_2$, $6H_2O$ | 500 | mg |
| $K_2SO_4$ | 450 | mg |
| $FeSO_4$, $7H_2O$ | 18 | mg |
| $CaCl_2$, $2H_2O$ | 2 | mg |
| NaCl | 500 | mg |
| KCl | 5000 | mg |
| Solution d'oligo-éléments (cf. Tableau II) | 1 | ml |
| Glycérol | 10000 | mg |

Ajuster le pH à 7,2 avec de la potasse concentrée de densité 1,38 et compléter à 1000 ml avec de l'eau ultra-purifiée. Effectuer une filtration stérilisante sur membrane de 0,2 μm.

Solution de glycérol

Dissoudre 50 g de glycérol dans une quantité adéquate d'eau ultra-purifiée et ajuster le volume à 1000 ml avec le même solvant. Effectuer une filtration stérilisante sur membrane de 0,2 μm.

L'anti-mousse employé en cours de fermentation est le Struktol J 673® (Schill et Seilacher).

**MILIEU B**

La préparation du milieu B est identique à celle du milieu A, à la différence près, qu'il convient d'ajouter en plus le tampon (pH 7,2) : acide 3-morpholinopropanesulfonique (M.O.P.S.) après addition de l'agent anti-mousse.

*La quantité de NTA utilisée pour la préparation du milieu B, peut être remplacée par une quantité adéquate de N'[Tris-(hydroxyméthyl) méthyl] glycine (Tricine commercialisé par Fluka® qui est de 360 mg.

On refroidit alors le bouillon de culture à 25°C. On prélève 5 litres de culture, on centrifuge ( 11000 - 14000.g) pendant 15 à 20 mn. Le surnageant est mélangé avec 5 litres de culture. On répète l'opération de centrifugation. Le culot est éliminé et le surnageant est filtré sur membrane écran en polycarbonate (Nucléopore®) 0,2 μm. Le filtrat obtenu est concentré sur cartouche à fibres creuses Amicon®, seuil de coupure 30000 Da ou équivalent. On obtient ainsi une solution enrichie en N-acétylhéparosane de haute masse moléculaire.

On ajoute à la solution une quantité adéquate de chlorure de sodium pour avoir une solution de 0,5 M en NaCl, puis on ajoute 4 volumes d'éthanol. On laisse se former le précipité pendant 5 minutes à température ambiante. On centrifuge à 5000.g pendant 20 minutes. On reprend les culots de centrifugation dans l'éthanol, on agite la suspension obtenue, et on la laisse reposer pendant 1 heure à température ambiante. On répète les opérations de centrifugation et de mise en suspension. On centrifuge à nouveau à 5000.g pendant 20 minutes. On sèche les culots de centrifugation obtenus dans une étuve sous vide à 40°C pendant 24 heures. Le N-acétylhéparosane ainsi obtenu est un "N-acétylhéparosane de haute masse moléculaire purifié". On reprend le N-acétylhéparosane dans de l'eau stérile de manière à avoir une solution de N-acétylhéparosane de haute masse moléculaire de concentration 28 g/l.

## EXEMPLE 1

### Obtention et caractérisation de l'enzyme

### 1- OBTENTION D'UNE CULTURE D'ESCHERICHIA COLI (K5) SEBR 3282 CONTENANT L'ENZYME

Pour obtenir l'enzyme, une culture d'Escherichia coli (K5) souche SEBR 3282 est effectuée dans des conditions environnementales favorables à la formation d'enzyme. En effet, il est nécessaire d'utiliser un milieu de culture complexe tel qu'il est décrit ci-dessous. Toutefois, d'autres milieux équivalents peuvent être utilisés. Les milieux C et D dont la composition est indiquée dans le Tableau IV ci-après seront appelés par la suite "milieux complexes". On ensemence 400 ml du milieu D, avec la souche Escherichia coli SEBR 3282 (déposée auprès de la CNCM de l'Institut Pasteur, Paris France sous le N°I-1013) et on incube sous agitation pendant 2 h à 37°C. La préculture obtenue est alors transférée dans un fermenteur de 18,5l contenant 11 l du milieu C, et on incube pendant 4 h à 37°C et à un pH égal à 7,4, la pression partielle en oxygène étant maintenue à 40 mmHg par régulation de l'injection d'air (jusqu'à 20 l/min.) et de l'agitation. On ajoute alors du glucose en introduisant de façon continue une solution stérile contenant 600 g/l de glucose à raison de 250 ml/h pendant 8 h. On poursuit la culture dans les mêmes conditions de température, de pH et de pression partielle en oxygène pendant 10 h après l'arrêt de l'addition de glucose.

Le suivi de la densité optique (DO) à $\lambda$ = 600 nm du milieu de culture permet d'affirmer qu'il n'y a pas de croissance de la biomasse pendant les 12 dernières heures de la culture.

## TABLEAU IV

**Composition et préparation du milieu C et du milieu D**
**(MILIEUX COMPLEXES)**

**MILIEU C**

Le milieu C est préparé par la réunion des trois solutions stériles ci-dessous :

Solution N°1

dans 700 ml d'eau ultra-purifiée dissoudre dans l'ordre :

| | |
|---|---|
| Complexant : N'[Tris-(hydroxyméthyl) méthyl] glycine (Tricine commercialisé par Fluka®) | 360 mg |
| $FeSO_4$, $7H_2O$ | 280 mg |
| $CaCl_2$, $2H_2O$ | 6,7 mg |
| $MgCl_2$, $6H_2O$ | 1270 mg |
| $K_2SO_4$ | 500 mg |
| KCl | 5000 mg |
| Hydrolysat de caséine (source principale d'acides aminés) HY CASE SF® (commercialisé par Sheffield) | 25000 mg |
| Extrait de levure (commercialisé par Difco®) | 18000 mg |
| Solution d'oligo-éléments (cf. tableau II) | 1 ml |

Agent antimousse Struktol J673® (commercialisé par Schill et Seilacher) : quelques gouttes à la pipette Pasteur.

Ajuster le pH à 7,4 avec une solution de KOH (d = 1,38) et compléter à 850 ml avec de l'eau ultra-purifiée. Autoclaver le milieu 45 minutes à 120°C.

Solution N°2

Dans environ 40 ml d'eau ultra-purifiée, dissoudre 5 g de $K_2HPO_4$ puis ajuster à 50 ml avec le même solvant. Filtrer la solution obtenue à travers un filtre de porosité 0,2 µm.

Solution N°3

Dissoudre 20,7 g de glucose dans une quantité adéquate d'eau ultra-purifiée et ajuster le volume à 100 ml avec le même solvant. Autoclaver à 110°C pendant 30 minutes.

**MILIEU D**

La préparation du milieu D est identique à celle du milieu C, à la différence près, qu'il convient d'ajouter en plus 20 g de tampon pH = 7,2 : (acide 3-morpholinopropanesulfonique) après l'addition de l'agent anti-mousse.

On obtient ainsi une culture d'Escherichia coli (K5) contenant l'enzyme, objet de l'invention.

## 2- MISE EN EVIDENCE IN VIVO DE L'ENZYME EN FERMENTEUR

La mise en évidence in vivo de l'enzyme a été effectuée par la fragmentation provoquée d'une quantité de N-acétylhéparosane de haute masse moléculaire ajoutée à une suspension de culture présentant une activité éliminasique.

La solution aqueuse de N-acétylhéparosane de haute masse moléculaire obtenue au stade précédent est ajoutée dans un fermenteur de 18,5 litres à une culture d'Escherichia coli (K5), souche SEBR 3282 contenant l'enzyme, une fois que la culture est parvenue au plateau. Ceci est mis en évidence par le suivi de la DO à $\lambda$ = 600 nm (1 - Obtention d'une culture d'Escherichia coli (K5) SEBR 3282 contenant l'enzyme).

Des échantillons de suspension de culture ont été pris à 3, 5, 7, 9,75 et 13,5 heures après addition du N-acétylhéparosane exogène ainsi que juste après cette addition.

Etape A

Détermination de la répartition des masses moléculaires de N-acétylhéparosanes au cours de la fermentation

La détermination de la répartition des masses moléculaires des N-acétylhéparosanes contenus dans les différents échantillons à été effectuée par HPLC d'exclusion.

a- Conditions opératoires de HPLC d'exclusion

- Colonne: TSK G 3000 SW® (LKB) 7,5 x 300 mm constituée de billes de silice de diamètre 10 µm et de porosité 250 Å.
- Eluant : solution aqueuse de sulfate de sodium 0,5 M, filtrée sur 0,2 µm et dégazée.
- Débit : 1 ml/minute
- Détection UV à $\lambda$ = 205 nm
- L'étalonnage est effectué à l'aide d'une gamme d'oligosaccharides dérivés de l'héparine (10 mg) de masses moléculaires suivantes (Da) : 1320, 1880, 2440, 3410 4000, 4540, 5000, 5370, 5730, 6150, 6670, 7540, 8660, 10090, 11560, 12950, 14810, 17390, 22670.

b- Préparation des échantillons

A partir d'un prélèvement effectué sur la suspension de culture d'un fermenteur. Centrifuger à 7000.g pendant 2 minutes. Récupérer le surnageant et le filtrer sur membrane 0,2 µm, précipiter le filtrat en ajoutant 4 volumes d'éthanol absolu soit 80 % d'éthanol final, agiter au Vortex, attendre 5 minutes. Centrifuger à 4000.g pendant 5 minutes, éliminer le surnageant, reprendre le culot dans l'eau ultra-purifiée au volume initial, agiter au Vortex et dialyser sur boudin Spectra-Por® (Spectrum) de seuil de coupure 10000 Da pendant une nuit contre de l'eau ultra-purifiée.

Sous agitation magnétique à 4°C, ajouter une solution concentrée de NaCl telle que la concentration finale de la solution contenant le N-acétylhéparosane soit de 0,5 M. Précipiter en ajoutant 4 volumes d'éthanol absolu pour 1 volume de dialysat salé, agiter au Vortex. Attendre 5 minutes, centrifuger à 4000.g pendant 5 minutes, jeter le surnageant, reprendre le culot dans du tampon pipérazine 25 mM pH 3,5 de manière à se situer entre 5 et 10 g de N-acétylhéparosane/litre, agiter au Vortex fortement.

Déposer l'équivalent de 1 mg de N-acétylhéparosane sur une colonne de résine Q-Sepharose® préalablement équilibrée avec le Tampon Pipérazine, procéder au lavage de la colonne de résine par 2 volumes de Tampon Pipérazine. Laver avec 4-5 volumes d'eau ultra-purifiée, éluer avec 2 volumes de NaCl 0,5 M, recueillir l'éluat et précipiter à l'éthanol absolu comme ci-dessus.

Centrifuger à 4000.g pendant 5 minutes et reprendre le culot dans de l'eau ultra-purifiée de manière à se situer entre 5 et 10 g/l de N-acétylhéparosane.

Cette solution est prête pour la détermination de la répartition des masses moléculaires par chromatographie d'exclusion.

Les résultats correspondant aux échantillons analysés, sont donnés sous forme de profils chromatographiques dans la figure 1.

A partir de ces données, les fragments de N-acétylhéparosanes ont été divisés en trois classes :

- Fragments ayant une masse moléculaire supérieure à 100000 Da
- Fragments ayant une masse moléculaire comprise entre 5000 et 100000 Da
- Fragments ayant une masse moléculaire égale à environ 5000 Da

L'abondance relative de 3 classes de fragments dans le fermenteur au cours du temps, est représentée dans la figure 2.

L'examen de la figure 1 permet les conclusions suivantes:

Le N-acétylhéparosane de haute masse moléculaire de la culture et celui qui lui a été ajouté évoluent progressivement vers du N-acétylhéparosane majoritairement de faible masse moléculaire.

L'examen de la figure 2 permet les conclusions suivantes:

Il existe des étapes fugitives où du N-acétylhéparosane de masse moléculaire intermédiaire est mis en évidence, c'est-à-dire que toute la gamme des masses moléculaires est représentée au même moment. Toutefois, au bout de 24 heures, le N-acétylhéparosane de haute masse moléculaire introduit dans le fermenteur, est fragmenté en quasi totalité (80 %) en des fragments ayant une masse moléculaire d'environ 5000 Da.

La fragmentation s'effectue donc sur du N-acétylhéparosane exogène soit par des enzymes en solution, soit au contact d'enzymes membranaires.

Etape B

Vérification de la structure des N-acétylhéparosanes de faible masse moléculaire obtenus par RMN

Les spectres RMN du proton et du carbone $^{13}C$ des N-acétylhéparosanes obtenus après fragmentation enzymatique sont comparés à ceux du N-acétylhéparosane décrits par W. E. Vann (Eur. J. Biochem., 1981, 116, 359-364).

L'étude des spectres obtenus avec les N-acétylhéparosanes de faible masse moléculaire (environ 5000 Da), confirme l'identité chimique du produit au N-acétylhéparosane décrit par W.E. Vann. Il s'agit de chaînes de polymères constituées de structures répétées de β-D-glucuronyl-1,4-α-N-acétyl-D-glucosaminyl-(1,4).

De plus, ces mêmes spectres permettent de conclure que les fragments comportent à l'extrémité non réductrice un reste d'acide glucuronique ayant une double liaison entre les carbones 4 et 5.

Les résultats obtenus indiquent bien que l'enzyme responsable de la fragmentation est de type β-éliminase en raison de la présence d'une double laison à l'extrémité non réductrice du N-acétylhéparosane de faible masse moléculaire obtenu, cette extrémité étant un reste d'acide glucuronique.

Par ailleurs, cette enzyme permet d'obtenir à la fin de la culture, à partir d'un N-acétylhéparosane de haute masse moléculaire, des fragments ayant dans leur majorité des tailles identiques qui correspondent à une masse moléculaire d'environ 5000 Da. Donc l'enzyme de l'invention doit être capable d'intervenir au niveau de la macromolécule de N-acétylhéparosane à une distance donnée de l'extrémité de cette macromolécule. Cela permet d'obtenir des fragments constitués par un nombre de motifs "β-D-glucuronyl-1,4-α-N -acétyl-D-glucosaminyl-(1,4)" bien précis. Il s'agit donc d'une endo-β-éliminase.

## 3- LOCALISATION DE L'ENZYME - OBTENTION DES PREPARATIONS CONTENANT CETTE ENZYME

Les étapes principales représentées dans le Schéma 1, étapes qui ont permis d'obtenir des préparations contenant l'enzyme et notamment un lysat brut et une préparation membranaire ayant une activité enzymatique, sont illustrées ci-après.

Etape A

Obtention d'un lysat brut présentant une activité enzymatique. Evaluation de son activité

a- Préparation d'un lysat brut

Le lysat brut présentant une activité enzymatique a été préparer à partir du culot d'une suspension de culture d'Escherichia coli (K5) souche SEBR 3282 contenant l'enzyme (1-Obtention d'une culture d'Escherichia coli

(K5) souche SEBR 3282 contenant l'enzyme), de la manière suivante:

Après incubation, le milieu de culture est refroidi à 25°C puis soumis à une centrifugation (31000.g) pendant 15 minutes. A 200 g de culots humides, sont ajoutés 400 ml de tampon Tris-HCl 50 nM pH 8 et une quantité adéquate d'EDTA de façon à obtenir une concentration finale de 10 mM. Sont ajoutés ensuite 100 mg de lysozyme.

La réaction enzymatique opérée par le lysozyme est menée à température ambiante, le pH étant maintenu à 8,0. Quand le mélange est homogène, il est placé à 4°C pour une nuit. Après cette pause, le pH est réajusté à 7,5 et on procédé à un ajout de $MgCl_2$ à 20 mM final, puis de DNASE I® 15 mg au total.

b- Mise en évidence de la fragmentation produite par le lysat brut.

A partir du lysat brut décrit ci-dessus, une fragmentation in vitro d'un N-acétylhéparosane de haute masse moléculaire a été observée en procédant de la manière suivante:

Le pH du lysat brut est ajusté à 6,8. On en prélève 600 µl et on y ajoute 1 mg de N-acétylhéparosane de haute masse moléculaire purifié, tel qu'il a été décrit dans la PREPARATION. On incube ensuite à 37°C pendant 24 heures. Une fois la réaction terminée, il est procédé à la détermination de la répartition des masses moléculaires du N-acétylhéparosane obtenu.

La détermination de la répartition des masses moléculaires des échantillons a été effectuée par HPLC d'exclusion comme il est décrit ci-dessus (2-Mise en évidence in vivo de l'enzyme en fermenteur - Etape A).

Un témoin de N-acétylhéparosane de haute masse moléculaire sans lysat a aussi été traité dans les mêmes conditions. La comparaison des profils chromatographiques obtenus mettent en évidence qu'aucune fragmentation du témoin ne s'est produite (Figure 3 - traits discontinus). Il s'agit encore d'un N-acétylhéparosane de haute masse moléculaire, composé par des constituants ayant des masses moléculaires allant de 10000 à 100000 Da.

Par contre, une fragmentation est observée lorsque le N-acétylhéparosane de haute masse moléculaire est traité par le lysat brut. Sur le profil chromatographique, on observe des fragments ayant une masse moléculaire comprise entre environ 4000 et 8000 Da, avec un pic accumulant des fragments d'environ 5000 Da correspondant à l'essentiel du produit final (Figure 3 - traits continus).

L'activité enzymatique de type β-éliminase de la souche ,SEBR 3282 peut donc être exploitée en absence de corps bactériens, in vitro, à partir d'un simple lysat brut. Il est également étonnant que les fragments ne soient pas scindés en dessous d'une certaine taille, à l'opposé par exemple de ce qui est obtenu avec la lyase phagique spécifique de la souche d'Escherichia coli (K5) qui procède à la disparition totale des fragments de 5000 Da vers des entités beaucoup plus petites (Gupta D., Jann B. and Jann K.; FEMS Microbiology Letters 1983, 16, p 13-17).

Etape B

Obtention d'une préparation membranaire présentant une activité enzymatique - Evaluation de son activité

Le lysat brut obtenu à l'étape précédente est soumis à une centrifugation à 15000.g pendant 60 minutes. Le culot est éliminé et le surnageant est centrifugé à 105000.g pendant 60 minutes. Le culot contenant les membranes bactériennes est isolé et repris dans le volume de départ avec de l'eau ultra-purifée.

La mise en évidence de l'activité éliminasique de cette préparation membranaire s'effectue comme décrit à l'étape précédente en incubant un aliquot de cette préparation mis en contact avec du N-acétylhéparosane de haute masse moléculaire à un pH voisin de la neutralité et une température de 37°C. Après une nuit, un échantillon est prélevé et l'évaluation de la répartition de sa masse moléculaire est effectuée par HPLC en utilisant la méthode citée précédemment (2-Mise en évidence in vivo de l'enzyme en fermenteur - Etape A). Les résultats obtenus montrent que l'activité enzymatique endo-β-éliminasique se retrouve au niveau des membranes bactériennes. Cela n'exclut pas, comme l'indique le Schéma 1, de détecter une activité dans le surnageant de la suspension de la culture obtenu, après une centrifugation basse vitesse (15000.g). En effet, une lyse partielle des bactéries se produit spontanément, après la phase plateau, au sein de la suspension de la culture.

## 4- SOLUBILISATION DE L'ENZYME

4.1 - Solubilisation à l'aide de détergents

En raison des techniques de dosage de l'activité enzymatique qui nécessitent une enzyme fonctionnelle, les détergents considérés ont été choisis majoritairement parmi les non ioniques et non dénaturants (Shütte H., Kula M.R. ; Biotechnology and Applied Biochemistry (1990), 12, 559-620).

La solubilisation de l'enzyme sous forme membranaire a été effectuée à partir d'un culot membranaire, tel que décrit ci-dessus (3-Localisation de l'enzyme - Obtention des préparations contenant cette enzyme - Etape B).

Les culots membranaires sont repris dans une solution de détergents de même volume que le lysat brut de départ, puis, le mélange est laissé en contact une nuit à 4°C. Ensuite, il est soumis à une centrifugation à 105000.g pendant 1 heure. Le surnageant contenant les protéines est récupéré, dialysé et passé sur une colonne affine pour les détergents, selon les prescriptions du fournisseur.

La mise en évidence de l'activité des différentes préparations contenant l'enzyme sous forme soluble a été effectuée en incubant un aliquot des préparations de haute masse moléculaire à un pH voisin de la neutralité et à une température de 37°C.

Après 6 jours d'incubation, des échantillons sont prélevés et la répartition des masses moléculaires des N-acétylhéparosanes obtenus est évaluée par HPLC selon la méthode précédemment décrite. Les résultats de cette étude sont rassemblés dans le tableau V.

Une partie du culot membranaire, repris dans le tampon Tris-HCl 50 nM, pH 8, a servi de témoin. Ce dernier a été traité dans les conditions citées ci-dessus.

Le dosage des protéines totales a été effectué à l'aide de "BCA protein assay reagent" de Pierce®.

Les résultats indiqués dans le tableau V démontrent, que mis à part l'échantillon traité avec la guanidine thiocyanate, dans les conditions opératoires indiquées, les préparations d'enzyme solubilisées par des détergents sont actives sous réserve d'abaisser la concentration en détergent de la solution aqueuse, après solubilisation de l'enzyme.

En effet, le témoin après 6 jours d'incubation à 37°C ne présente qu'une faible attaque du N-acétylhéparosane, de l'ordre de 10 %, tandis que dans les autres essais, le N-acétylhéparosane fragmenté de faible masse moléculaire représente environ 90 % de la masse initiale, ce qui est un maximum.

**TABLEAU V**

| ESSAI | PROTEINES MEMBRANAIRE EN g/l SOUS FORME SOLUBLE | TAUX DE SOLUBILISATION*/PROTEINES MEMBRANAIRES | ACTIVITE ENDO-β-ELIMINASIQUE |
|---|---|---|---|
| **1. TEMOIN TRIS-HCL** | 0,32 | 17 % | FAIBLE |
| **2. TRITON X-100® 2%** | 0,88 | 44,5 % | FORTE |
| **3. TRITON X-114® 2%** | 1,64 | 68,6 % | FORTE |
| **4. DOC 2%** | 0,86 | 55,1 % | FORTE |
| **5. NP-4O® 2%** | 0,97 | 58,0 % | FORTE |
| **6. GUANIDINE-THIOCYANATE 4M** | 2,72 | 90,7 % | NULLE |
| **7. TWEEN 80® 2%** | 0,57 | 24,1 % | FORTE |
| **8. GUANIDINE-HCL 0,1 M** | 1,04 | 58,4 % | FORTE |

Un autre fait intéressant qui apparaît sur la superposition des profils chromatographiques, est le déplacement du pic accumulant les fragments de N-acétylhéparosane majoritairement de basses masses moléculaires de 5000 Da. Pour l'enzyme membranaire (témoin) il se situe vers 5000 Da. Pour l'échantillon traité au Triton X-114®, il se situe vers 6500 à 7000 Da (selon standard héparine).

L'étude de la solubilisation de l'enzyme permet de conclure que:

- l'enzyme peut-être solubilisée par un grand nombre de détergents et obtenue sous forme active;

- la forme soluble de l'enzyme permet d'obtenir des pics rassemblant une majorité de fragments de N-acétylhéparosane autour d'une masse moléculaire (sommet du pic) supérieure à celle constatée pour la forme membranaire de l'enzyme.

4.2- Solubilisation par lyse alcaline

La solubilisation de l'enzyme par lyse alcaline a été effectuée à partir d'un culot bactérien brut. Le culot bactérien brut a été obtenu par centrifugation du milieu de culture à 10000.g pendant 10 minutes (3- Localisation de l'enzyme - Obtention des préparations contenant cette enzyme - Etape A).

Le culot bactérien a été congelé à -20°C. 180 g de ce culot bactérien sont mis en suspension dans 100 ml d'eau ultra-purifée et le pH de la suspension est ajusté à 11 à l'aide d'une solution d'hydroxyde de sodium concentrée. La suspension alcaline est maintenue à pH 11 et à 4°C pendant 2 heures. Le lysat ainsi obtenu est ultra-centrifugé à 105000.g pendant une heure. Le surnageant est récupéré le pH est ajusté à 7, puis dialysé à 4°C pendant une nuit dans un boudin de point de coupure 10000 contre 10 litres d'eau ultra-purifiée (Débit d'eau 90- 120 ml/heure) Une partie du lysat ainsi obtenu est conservé à 4°C et une autre à -80°C.

Pour déterminer l'activité enzymatique, 500 µl de N-acétylhéparosane de haute masse moléculaire (PREPARATION) ont été incubés pendant 15 heures à 37°C avec 125 µl du lysat obtenu et conservé soit à 4°C soit à -80°C. La détermination de la masse moléculaire de N-acétylhéparosane obtenu après incubation, a démontré que l'activité du lysat obtenu par lyse alcaline, est forte. Les conditions de conservation ne semblent pas avoir un rôle important puisque la même activité a été observée avec le lysat conservé à 4°C, et avec celui conservé à -80°C.

L'étude de la solubilisation de l'enzyme permet de conclure que :

- la solubilisation de l'enzyme par lyse alcaline et ultracentrifugation permet d'obtenir une activité de type β-éliminase du même ordre que celle résultant de l'action des détergents.
- la congélation transitoire du lysat à -80°C n'entraîne aucune perte de l'activité enzymatique.

## 5- DETERMINATION DU POINT ISOELECTRIQUE (pHi) DE L'ENZYME SOLUBILISEE

La détermination du pHi de l'éliminase se fait grâce à la méthode "chromatofocusing". Son principe est de créer, à travers une colonne de chromatographie, un gradient de pH. Les protéines sont alors éluées quand leurs charges sont globalement neutres (à leur pHi). La zone de pH testée est comprise entre pH 3,5 et pH 9.

a- Matériel utilisé

- Colonne basse pression : 10 x 30 cm (Pharmacia®)
- Gel PBE 94 (Pharmacia®)
- Eluant : "Polybuffer 96 et Polybuffer 74 (Pharmacia®)
- Tampon de départ pour 6 < pH < 9 : diéthanolamine-HCl 0,025 M à pH 9,5
- Tampon de départ pour 3,5 < pH < 6 : histidine-HCl 0,025 M à pH 6,2
- Pompe : Gilson®

b- Obtention d'éliminase solubilisée

4 g de culots membranaires humides sont introduits dans 10 ml de tampon Tris-HCl 0,1 M à pH 10,5, à 0,3 M de $CaCl_2$. L'opération de la solubilisation dure 2 heures à température ambiante. La suspension est alors centrifugée à 105000.g pendant 1 heure. Le surnageant contenant l'enzyme est à 3 mg/ml de protéines actives.

Dans un premier temps, la zone de pH choisie se situait entre pH 6 et 9. Des résultats plus précis ont été obtenus en répétant les essais et en utilisant une zone de pH comprise entre 4,7 et 5,4.

L'activité enzymatique des différentes fractions a été évaluée en mettant en contact les fractions obtenues lors de la chromatographie en gradient avec un N-acétylhéparosane de haute masse moléculaire et en déterminant la distribution des masses moléculaires du N-acétylhéparosane obtenu après incubation pendant 60 heures à 37°C et à pH 6,8.

Le pHi de l'enzyme est compris entre pH 4,7 et pH 5,4.

Des expériences de "chromatofocusing" complémentaires ont été entreprises pour mieux préciser le pHi de l'enzyme. Le maximum de l'activité a été trouvé dans une fraction de pH 5,1. Le pHi de l'éliminase est par conséquent de 5,1 (figure 4).

## 6- DETERMINATION DE LA MASSE MOLECULAIRE DE L'ENZYME SOLUBILISEE

Essai 1

La détermination de la masse moléculaire de l'éliminase a été effectuée par chromatographie en perméation de gel (GPC). Les protéines sont éluées en fonction de leur masse moléculaire, les plus petites tailles sortant en premier.

a- Matériel utilisé

- Colonne TSK G 2000 SW® de 7,5 x 300 mm
- Eluant NaCl 0,3 M Tris 50mM pH 7
- Débit 0,3 ml/minute
- Détection à 280 nm avec une sensibilité de 0,2 AUFS

b- Obtention d'éliminase solubilisée

4 g de culots membranaires humides sont introduits dans 10 ml de tampon Tris-HCl 0,1 M à pH 10,5, à 0,3 M de chlorure de calcium. La solubilisation dure 2 heures à température ambiante La suspension est alors centrifugée à 105000.g pendant 1 heure. Le surnageant contenant l'enzyme est à 3 mg/ml de protéines actives. 24 fractions, collectées toutes les 30 secondes, ont été obtenues. L'activité enzymatique des différentes fractions obtenues a été évaluée en mettant en contact chaque fraction avec un N-acétylhéparosane de haute masse moléculaire. Après incubation pendant 38 heures à 37°C et à pH 7, la distribution des masses moléculaires du N-acétylhéparosane obtenu a été déterminée. Les fractions les plus actives sont situées entre 22 et 23 minutes (figure 5a). En fonction des deux pics les plus élevés en activité éliminasique, la masse moléculaire de l'éliminase est située entre 62000 et 70000 Da et plus précisément vers 65000 Da. On peut donc dire dans un premier temps que l'éliminase à un poids moléculaire situé entre 62000 et 70000 Da, et plus particulièrement vers 65000 Da.

Essai 2

Pour mieux préciser la masse moléculaire de l'enzyme, un essai supplémentaire a été réalisé en utilisant une colonne chromatographique, colonne TSK G 3000 SW® de 7,5 x 300 mm. Cette colonne permet une meilleure séparation des masses moléculaires d'environ 100000 Da. La technique utilisée et les conditions opératoires sont identiques à celles décrites dans l'essai 1. L'étalonnage inclut des masses moléculaires de 43000 Da et 67000 Da. Lors de cet essai, on constate que les temps de rétention des fractions d'activité maximale de l'enzyme, correspondent à des masses moléculaires de 65000 Da à 66000 Da (± 1500 Da) (Figure 5b).

## 7- FACTEURS ENVIRONNEMENTAUX EXERÇANT UNE INFLUENCE SUR L'ACTIVITE DE L'ENZYME - PREPARATION MEMBRANAIRE

Pour cette étude, une préparation membranaire telle que décrite ci-dessus (3- Localisation de l'enzyme - Etape B - Obtention d'une préparation membranaire présentant une activité enzymatique. Evaluation de son activité) a été utilisée. L'activité enzymatique de cette préparation, observée selon les différents paramètres étudiés, à été évaluée par la détermination de la dispersion des masses moléculaires d'un N-acétylhéparosane de haute masse moléculaire soumis à son action.

a- Température

La température optimale de fonctionnement de l'enzyme est voisine de 37°C, sa température d'inactivation est de 60°C et à 20°C, l'enzyme conserve 40 % de son activité versus celle à 37°C.

b- pH

La zone optimale de fonctionnement de l'enzyme se situe entre les valeurs pH 6 et pH 7. A pH 5 et à pH 7,5, l'enzyme conserve 40 % de son activité versus celle observée dans la zone de pH 6-7. A pH 8,5, l'enzyme conserve moins de 10 % de cette activité.

c- Effet des ions monovalents ($Na^+$)

La zone optimale de concentrations en ions sodium pour le fonctionnement de l'enzyme se situe au voisinage de 0,2 M. A cette concentration en ions monovalents, l'activité de l'enzyme augmente d'environ 15 % par rapport au témoin. Il faut noter ici que les ions sodium permettent de déplacer le sommet du pic des fragments de N-acétylhéparosane vers les hautes masses moléculaires. L'enzyme membranaire, placée dans ces conditions, engendre des fragments de masse moléculaire supérieure d'environ 500 Da par rapport à ceux du témoin.

Certains ions monovalents, le sodium en particulier, permettent donc de moduler la taille des fragments obtenus en final.

d- Effet des ions divalents ($Ca^{2+}$)

La zone optimale de concentrations en ions calcium pour le fonctionnement de l'enzyme se situe au voisinage de 0,2 M. Pour cette concentration, l'activité de l'enzyme augmente d'environ 50 % par rapport au témoin.

e- Constante de Michaelis

La mesure de la réaction de fragmentation en fonction de la concentration en substrat (ici le N-acétylhéparosane de haute masse moléculaire) permet après tracé selon Lineweaver-Burk de constater :

- que l'on obtient une droite ; l'enzyme répond donc à une cinétique Michaelienne ;
- que la constante de Michaelis est égale à la concentration de substrat pour laquelle la vitesse de réaction est la moitié de la vitesse maximale et est pratiquement égale à 1 g de N-acétylhéparosane/litre de solution réactionnelle.
- que de fortes concentrations en substrat, par exemple 30 g/l, à partir desquelles la solution de N-acétylhéparosane commence à devenir visqueuse, ne constituent pas un obstacle sensible au bon fonctionnement de l'enzyme.

f- Stabilité de l'enzyme

*Influence du pH :*

Après passage à pH 10,5 où l'enzyme est inactivée, l'enzyme récupère son activité une fois revenue à des valeurs pH proches de la neutralité. L'enzyme supporte également un passage à pH 3,5.

*Influence de la température - Conservation à 4°C :*

Après stockage à 4°C pendant 3 mois, aucune perte significative d'activité n'est constatée sur l'enzyme.

*Influence d'un agent réducteur :*

La présence de dithio érythritol (DTE), exerce une influence stabilisante sur l'activité de l'enzyme.

## 8- FACTEURS ENVIRONNEMENTAUX EXERÇANT UNE INFLUENCE SUR L'ACTIVITE DE L'ENZYME - CULOT BACTERIEN BRUT

Le culot bactérien brut, qui peut être obtenu après centrifugation du milieu de culture à 10000.g pendant 10 minutes, peut aussi être utilisé pour fragmenter le N-acétylhéparosane de haute masse moléculaire, à des

fins préparatoires. Les conditions d'utilisations de culots bactériens bruts, induits au préalable pour avoir une activité enzymatique dans le but de fragmenter le N-acétylhéparosane de haute masse moléculaire, ont été étudiées.

a- Température

La température d'activité enzymatique maximale du culot est voisine de 40°C. Le pH interfère légèrement avec la température sur ce produit.

b- pH

La zone optimale d'activité enzymatique du culot se situe entre les valeurs pH 6,6-6,8. A pH 7,5, l'enzyme conserve 40 % de son activité maximale.

c- Effet des ions monovalents ($Na^+$)

Parmi les ions monovalents étudiés $Na^+$, $K^+$ et $Li^+$ sous forme de sels (chlorures), l'ion $Na^+$ exerce le plus grand effet activateur sur l'activité enzymatique du culot bactérien brut. L'effet activateur maximal est observé lorsque la concentration en ions $Na^+$, est d'environ 0,3 M.

d- Effet des ions divalents ($Ca^{2+}$)

Parmi les ions divalents étudiés $Ca^{2+}$ et $Mg^{2+}$ sous forme de sels (chlorures), l'ion $Ca^{2+}$ exerce le plus grand effet activateur sur l'activité enzymatique du culot bactérien brut. La zone optimale de concentration en ions calcium, se situe à 0,1-0,15 M. Dans ces conditions l'activité de l'enzyme est multipliée par deux.

Des ions divalents métalliques comme $Zn^{2+}$ et $Cu^{2+}$ se sont révélés plus ou moins inhibiteurs de cette activité enzymatique.

e- Constante de Michaelis

En raison des caractéristiques de la préparation brute, l'étude cinétique réalisée sur l'enzyme liée aux corps bactériens, est interprétée avec réserve. Toutefois, il a été constaté que :

- l'activité enzymatique du culot bactérien brut montre un comportement de type Michaelien, comme l'enzyme obtenue sous forme membranaire (préparation membranaire) ;
- la constante de Michaelis dans les conditions optimales (température, pH, concentration en ions $Ca^{2+}$), est de l'ordre de 2,2 à 3,2 g en N-acétylhéparosane par litre de solution réactionnelle.
  La constante de Michaelis pour le culot bactérien brut est donc nettement supérieure à celle trouvée pour la préparation membranaire.

f- Inhibiteurs compétitifs de l'interaction enzyme / substrat

Il a été observé que la N-acétylglucosamine et l'acide glucuronique, tous les deux composants du N-acétylhéparosane, sont des inhibiteurs compétitifs de l'activité enzymatique du culot bactérien brut.

g- Modification du substrat

Les groupements carboxylique et N-acétyle du N-acétylhéparosane ont été éliminés par le 1-éthyl-3-(3-diméthylaminopropyl) carbodiimide hydrochloride (EDC) et la soude à chaud. Ce polymère, privé de l'un ou de l'autre de ces groupements, n'est plus attaqué par l'enzyme.

h- Stabilité de l'activité enzymatique du culot

*Influence de la température :*

Le culot bactérien peut être stocké à -20°C ou 80°C, sans perte notable de son activité éliminasique.

## 9- ETUDE DE LA CINETIQUE DE FRAGMENTATION DU N-ACETYLHEPAROSANE PAR L'ENZYME - CULOT BACTERIEN BRUT

Pour étudier la cinétique de fragmentation du N-acétylhéparosane par l'enzyme contenue dans le culot bactérien brut, un échantillon de N-acétylhéparosane de haute masse moléculaire a été mis en contact avec un culot bactérien d'Escherichia coli (K5) SEBR 3282, riche en éliminase, à 40°C en présence de 140 mM de $CaCl_2$ et de 50 mM de tampon Bis-tris-propane pH 6,6. A la fin de la réaction enzymatique, il a été observé une fragmentation du N-acétylhéparosane de l'ordre de 90 %. En effet, 90 % du N-acétylhéparosane de haute masse moléculaire a été transformé en héparosane de faible masse moléculaire. Le N-acétylhéparosane fragmenté a été récupéré, mis à nouveau en contact avec un culot bactérien brut "frais", et incubé de nouveau à 37°C. A la fin de cette opération, un échantillon de N-acétylhéparosane a été prélevé et purifié selon les conditions décrites dans "1-Obtention d'une culture d'Escherichia coli (K5) SEBR 3282 contenant l'enzyme -Etape A - Détermination de la répartition des masses moléculaires de N-acétylhéparosane au cours de la fermentation ; b- Préparation des échantillons".

Les masses moléculaires de cet échantillon ont été ensuite évaluées par HPLC d'échange d'ions.

Conditions opératoires d'HPLC d'échange d'ions

HPLC pour réaliser des gradients binaires (2 pompes)
- Colonne Pharmacia® Mono Q® HR 5/5 de volume 1 ml
- Détecteur UV à 220 nm
- Eluant :

  Eau ultra-purifiée (A)

  Solution aqueuse de NaCl 0,5 M (B)

  Utiliser un gradient :

| TEMPS (MIN.) | % DE B |
|---|---|
| 0 → 2.00 | 30.00 |
| 15.00 | 60.00 |
| 17.00 | 100.00 |
| 19.00 | 100.00 |
| 20.00 → 25.00 | 30.00 |

- Débit : 1 ml/minute
- L'étalonnage est effectué à l'aide d'une gamme d'oligosaccharides dérivés du N-acétylhéparosane, constitués de 6 à 14 unités disaccharidiques.

Les résultats obtenus ont confirmé que la fragmentation de N-acétylhéparosanes de haute masse moléculaire en N-acétylhéparosanes de faible masse moléculaire, est quasi totale (≧ 90 %).

Les pics majoritaires des fragments constituant le N-acétylhéparosane de faible masse moléculaire correspondent à des fragments de 8 à 12 unités disaccharidiques.

La masse moléculaire des fragments du N-acétylhéparosane de faible masse moléculaire n'est donc pas modifiée par un nouvel apport d'éliminase. Ces résultats confirment que l'enzyme permet d'effectuer une fragmentation quasi complète d'un N-acétylhéparosane de haute masse moléculaire et d'obtenir des fragments ayant une masse moléculaire d'environ 5000 Da ou légèrement inférieure.

**EXEMPLE 2**

**Fragmentation modulée d'un N-acéthylhéparosane de haute masse moléculaire- Obtention d'un N-acétylhéparosane de masse moléculaire d'environ 7000 Da**

Stade A

Mélanger 25 g de N-acétylhéparosane de haute masse moléculaire, obtenu selon la PREPARATION avec une préparation d'enzyme solubilisée par un détergent ou encore par lyse alcaline (Exemple 1 ; 4- Solubilisation de l'enzyme ; 4.2 Solubilisation par lyse alcaline) obtenue à partir de 500 g de culot bactérien frais, lui-même obtenu selon l'Exemple 1 (1- Obtention d'une culture d'Escherichia coli (K5) SEBR 3282, contenant l'enzyme). Le pH est ajusté à 7 à l'aide du tampon Tris-HCl 0,05 M pH 7.

Ajouter une quantité adéquate de $CaCl_2$ de façon à obtenir une concentration finale de 0,2 M. Incuber à 37°C pendant 24 heures.

Stade B

Centrifuger le mélange obtenu pendant 5 minutes à 4000.g. Récupérer le surnageant et le filtrer sur membrane 0,2 μm. Précipiter le filtrat en ajoutant 4 volumes d'éthanol absolu soit 80 % d'éthanol final. Centrifuger à 4000.g pendant 5 minutes, éliminer le surnageant, reprendre le culot dans l'eau ultra-purifée. Dialyser sur boudin Spectra-Por® (Spectrum) de seuil de coupure 10000 Da pendant une nuit contre de l'eau ultra-purifée.

Sous agitation magnétique à 4°C, ajouter une solution concentrée de chlorure de sodium telle que la concentration finale soit de 0,5 M avant de précipiter en ajoutant 4 volumes d'éthanol absolu pour 1 volume de dialysat sale. Centrifuger à 4000.g pendant 5 minutes, jeter le surnageant.

Reprendre les culots de centrifugation, dans un tampon appelé tampon D, de composition Tris-HCl 20 mM pH 7,5, à raison de 100 ml/g. La solution obtenue est chromatographiée sur une colonne d'échange d'anions forts comportant une matrice d'agarose réticulée avec des groupes ammonium quaternaires (le "Q-Sepharose fast flow" de Pharmacia®) préalablement équilibré avec le tampon D, à raison de 50 ml de gel pour 1 g de poudre. Laver le gel avec une quantité suffisante de tampon D pour le retour à la ligne de base de la détection UV à 214 nm, puis avec une solution de pipérazine 25 mM dont le pH a été ajusté à 3,5. Eluer avec une solution de pH 3,5 ayant une composition : NaCl 0,5 M et pipérazine 25 mM. L'éluat est neutralisé à l'aide d'une solution de NaOH 5 N.

Précipiter en ajoutant 4 volumes d'éthanol. Centrifuger à 4000.g pendant 5 minutes. Récupérer le culot de centrifugation et sécher. On obtient ainsi un N-acétylhéparosane constitué de chaînes dont la majorité à une masse moléculaire comprise entre 6000 et 8000 Da.

**EXEMPLE 3**

**Fragmentation modulée du N-acétylhéparosane de haute masse moléculaire fixé sur colonne de résine échangeuse d'anions en présence de l'enzyme, objet de la présente invention.**

Une masse de résine Q-Sepharose® (Pharmacia) est dans un premier temps saturée avec du N-acétylhéparosane majoritairement de haute masse moléculaire comme décrit dans la PREPARATION. On introduit ensuite une préparation d'enzyme membranaire en solution dans un tampon Tris-HCl pH 7,2 et contenant du chlorure de sodium à 0,25 M. Cette dernière solution a été préparée à partir d'un lysat brut comme décrit dans l'EXEMPLE 1 (3- Localisation de l'enzyme. Obtention des préparations contenant cette enzyme - Etape B), mais en utilisant un tampon Tris-HCl pH 7,2 contenant du chlorure de sodium à 0,25 M au lieu de l'eau ultra-purifiée.

On laisse réagir pendant 60 heures. Le N-acétylhéparosane relargué en raison de la présence de NaCl 0,25 M est isolé et analysé. Il s'agit d'un N-acétylhéparosane dont la majorité des chaînes à un poids moléculaire compris entre 3200 et 4000 Da. Sur le profil chromatographique représentant la distribution des masses moléculaires, le maximum se situe à 3600 Da.

Le N-acétylhéparosane fixé sur la résine est ensuite élué en utilisant une solution aqueuse de NaCl à 0,30 M. L'étude du profil chromatographique représentant la distribution des masses moléculaires permet de confirmer que le maximum se situe à 4300 Da. Il s'agit d'un N-acétylhéparosane constitué de chaînes plus longues par rapport à celles du N-acétylhéparosane relargué.

Selon une autre variante, il est possible de procéder comme ci-dessus mais en utilisant une préparation d'enzyme membranaire en solution dans un tampon Tris-HCl pH 7,2 et contenant du chlorure de sodium à 0,5

M. Dans ce cas, l'étude du profil chromatographique de la distribution des masses moléculaires du N-acétylhéparosane relargué permet de confirmer qu'il s'agit d'un N-acétylhéparosane constitué de chaînes plus longues, le maximum du pic se situant à environ 9200 Da. Cela démontre qu'en conjonction avec une résine échangeuse d'anions, on peut moduler la fragmentation du N-acétylhéparosane en utilisant des préparations membranaires ayant une concentration en NaCl donnée.

## AUTRES EXEMPLES DE LA MISE EN OEUVRE DE L'ENZYME POUR LA FRAGMENTATION D'UN N-ACETYLHEPAROSANE DE HAUTE MASSE MOLECULAIRE

La mise en oeuvre de l'enzyme, objet de la présente invention lors de la fragmentation d'un N-acétylhéparosane de haute masse moléculaire a aussi été illustrée dans l'exemple 1 et notamment dans :

* 2- Mise en évidence in vivo de l'enzyme en fermenteur.
* 3- Localisation de l'enzyme. Obtention des préparations contenant cette enzyme.
  Etape A - Obtention d'un lysat brut présentant une activité enzymatique. Evaluation de son activité ; b) mise en évidence de la fragmentation produite par un lysat brut, et
  Etape B - Obtention d'une préparation membranaire présentant une activité enzymatique. Evaluation de son activité.
* 4- Solubilisation de l'enzyme

Il s'agit de procédés de fragmentation d'un N-acétylhéparosane de haute masse moléculaire qui sont aussi applicables au niveau industriel ou semi-industriel.

## Revendications

**1-** Enzyme capable de fragmenter un N-acétylhéparosane de haute masse moléculaire caractérisée en ce que :

- elle est obtenue d'une souche d'Escherichia coli (K5) souche SEBR 3282 ou d'un mutant spontané ou induit de cette souche,
- sa masse moléculaire est comprise entre 62000 et 70000 Da,
- son point isoélectrique se situe dans la zone de pH comprise entre 4,7 et 5,4 unités pH,
- elle est une éliminase, plus précisément une endo-β-éliminase.

**2-** Enzyme selon la revendication 1 caractérisée en ce que :

- elle est d'origine membranaire,
- la température de son fonctionnement optimal (activité maximale) est voisine de 37°C et la température à laquelle elle est inactivée est environ 60°C,
- la zone optimale de pH pour son fonctionnement optimal se situe entre les valeurs de pH 6 et 7,
- la zone optimale de concentration en ions monovalents ou divalents pour son fonctionnement optimal, se situe au voisinage de 0,2 M.

**3-** Enzyme selon les revendications 1 ou 2, caractérisée en ce que :

- sa masse moléculaire est comprise entre 65000 et 66000 Da (± 1500 Da),
- son point isoélectrique est de 5,1 unités pH,
- la zone optimale de pH pour son fonctionnement optimal, se situe entre pH 6,6 et pH 6,8,
- pour son fonctionnement, la zone optimale de concentration en ions monovalents se situe à 0,25 M et la zone optimale de concentration en ions divalents, se situent à 0,15 M.

**4-** Procédé d'obtention de l'enzyme selon la revendication 1, sous forme de culots bactériens bruts la contenant, caractérisé en ce que l'on effectue une culture d'Escherichia coli (K5) SEBR 3282 ou d'un mutant spontané ou induit de cette souche, dans un milieu de culture favorable à la formation de cette enzyme, et que l'on isole le culot de la culture par centrifugation.

**5-** Préparation contenant l'enzyme selon la revendication 1, constituée d'un culot bactérien brut obtenu d'une culture d'Escherichia coli (K5) SEBR 3282 ou d'un mutant spontané ou induit de cette souche, contenant cette enzyme.

**6-** Procédé d'obtention de l'enzyme selon la revendication 1, sous forme de lysats bruts la contenant, caractérisé en ce que l'on effectue une culture d'Escherichia coli (K5) SEBR 3282 ou d'un mutant spontané ou induit de cette souche, dans un milieu de culture favorable à la formation de cette enzyme, que l'on isole le culot de la culture et que l'on soumet ce culot à une lyse.

**7-** Préparation contenant l'enzyme selon la revendication 1, constituée d'un lysat brut, obtenu par lyse d'un culot bactérien brut contenant cette enzyme.

**8-** Préparation contenant l'enzyme selon la revendication 1, constituée d'une préparation membranaire obtenue par lyse d'un culot bactérien contenant cette enzyme et, ensuite par séparation des membranes du lysat du culot bactérien.

**9-** Préparation de l'enzyme selon la revendication 1, contenant cette enzyme sous forme solubilisée.

**10-** Préparation selon la revendication 9, dans laquelle l'enzyme a été solubilisée par des détergents anioniques ou cationiques.

**11-** Préparation selon la revendication 9, dans laquelle l'enzyme a été solubilisée par lyse partielle en milieu alcalin d'un culot bactérien brut contenant cette enzyme.

**12-** Procédé de fragmentation de N-acétylhéparosane de haute masse moléculaire mettant en oeuvre l'enzyme selon la revendication 1.

**13-** Procédé pour la préparation de N-acétylhéparosanes ayant des masses moléculaires regroupées, se situant entre 2000 et 1000 Da, caractérisé en ce que l'on traite un N-acétylhéparosane de haute masse moléculaire (polysaccharide K5) avec une préparation contenant l'enzyme selon la revendication 1, en présence d'une solution de chlorure de sodium.

**14-** Procédé pour la préparation de N-acétylhéparosanes selon la revendication 13, caractérisé en ce que l'on traite un N-acétylhéparosane de haute masse moléculaire avec une préparation contenant l'enzyme selon la revendication 1, en présence d'une solution de chlorure de sodium ayant une molarité de 0,2 à 0,5 M.

**15-** Procédé selon la revendication 13, caractérisé en ce que l'héparosane de haute masse moléculaire est fixé sur une colonne de résine échangeuse d'ion sur laquelle on fait passer une préparation d'enzyme selon la revendication 1 sur la colonne et que l'on élue avec une solution de chlorure de sodium de 0,2 à 0,5 M.

**16-** Procédé selon l'une quelconque des revendications 12 à 15, caractérisé en ce que la réaction enzymatique est effectuée entre pH 4,0 et pH 9,0, de préférence entre pH 6,4 et pH 7,4.

**17-** Procédé selon la revendication 13, caractérisé en ce que la résine est du type échangeuse d'anions.

**18-** Procédé selon la revendication 12, caractérisé en ce que l'enzyme selon la revendication 1, est utilisée sous forme immobilisée.

**19-** Préparation contenant l'enzyme selon la revendication 1, en association avec une autre enzyme ou des substances organiques ou minérales.

**20-** Enzyme selon la revendication 1, caractérisée en ce qu'elle peut être obtenue à partir la souche d'Escherichia coli (K5), Bi 8337-41 (010:K5:H4).

**21-** Utilisation de l'enzyme selon l'une des revendications 1 à 4, pour la fragmentation de N-acétylhéparosanes de haute masse moléculaire.

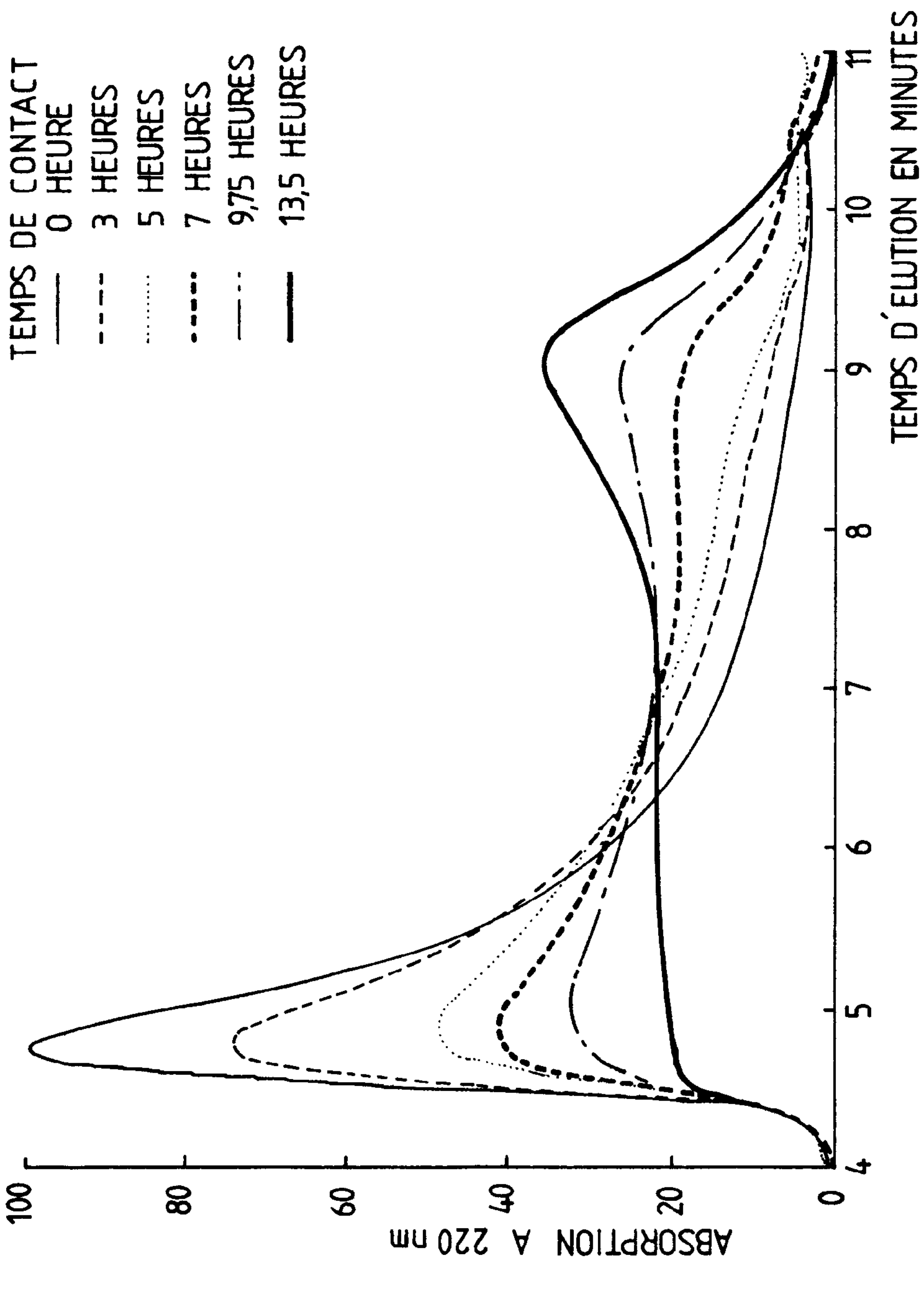


FRAGMENTATION IN-VIVO EN FERMENTEUR
AUX TEMPS 0;3;5;7;9,75 ET 13,5 HEURES

FIG.1

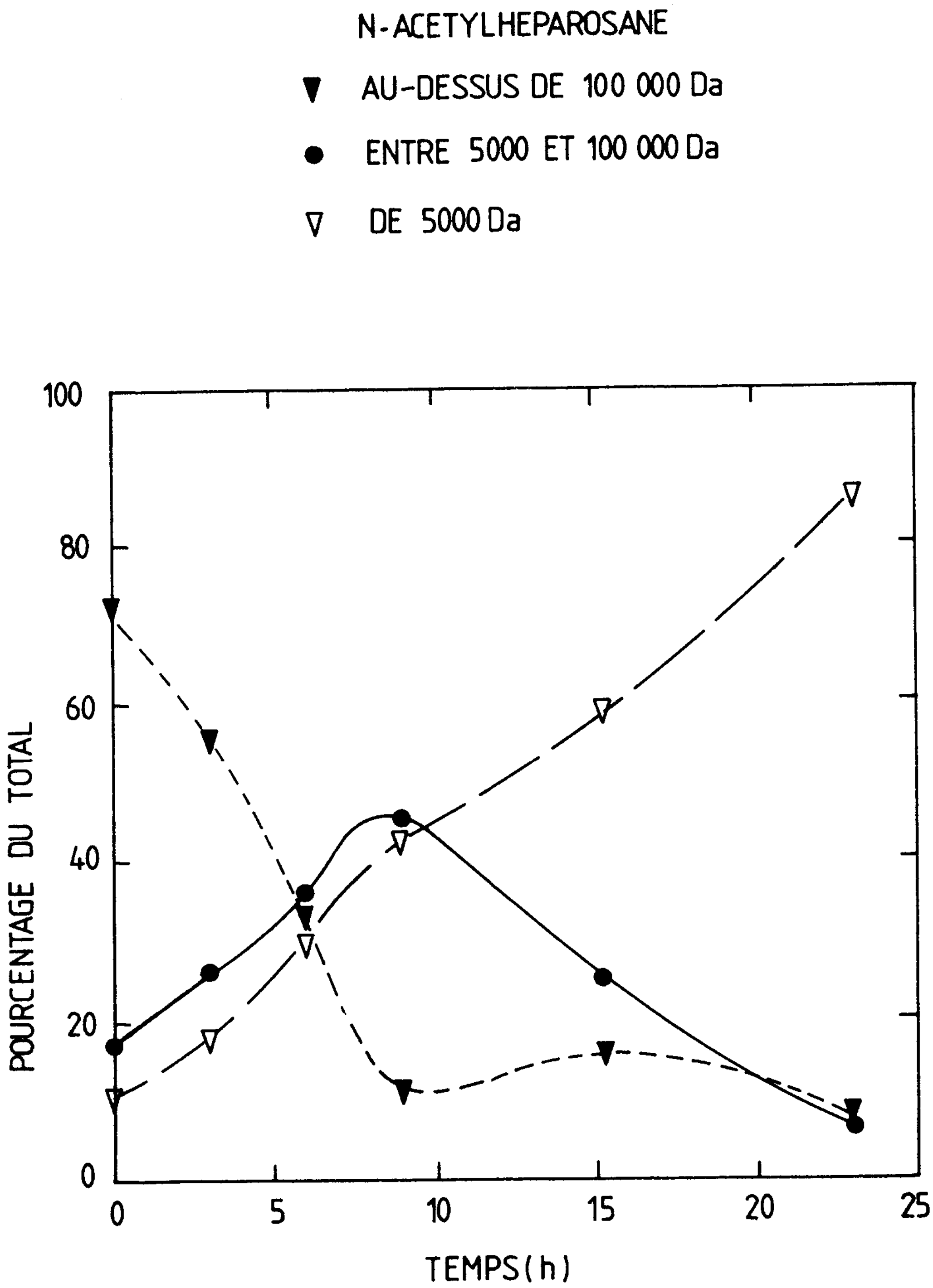


ABONDANCE RELATIVE DES DIFFERENTES CLASSES DE N-ACETYLHEPAROSANE EN FONCTION DU TEMPS.

FIG. 2

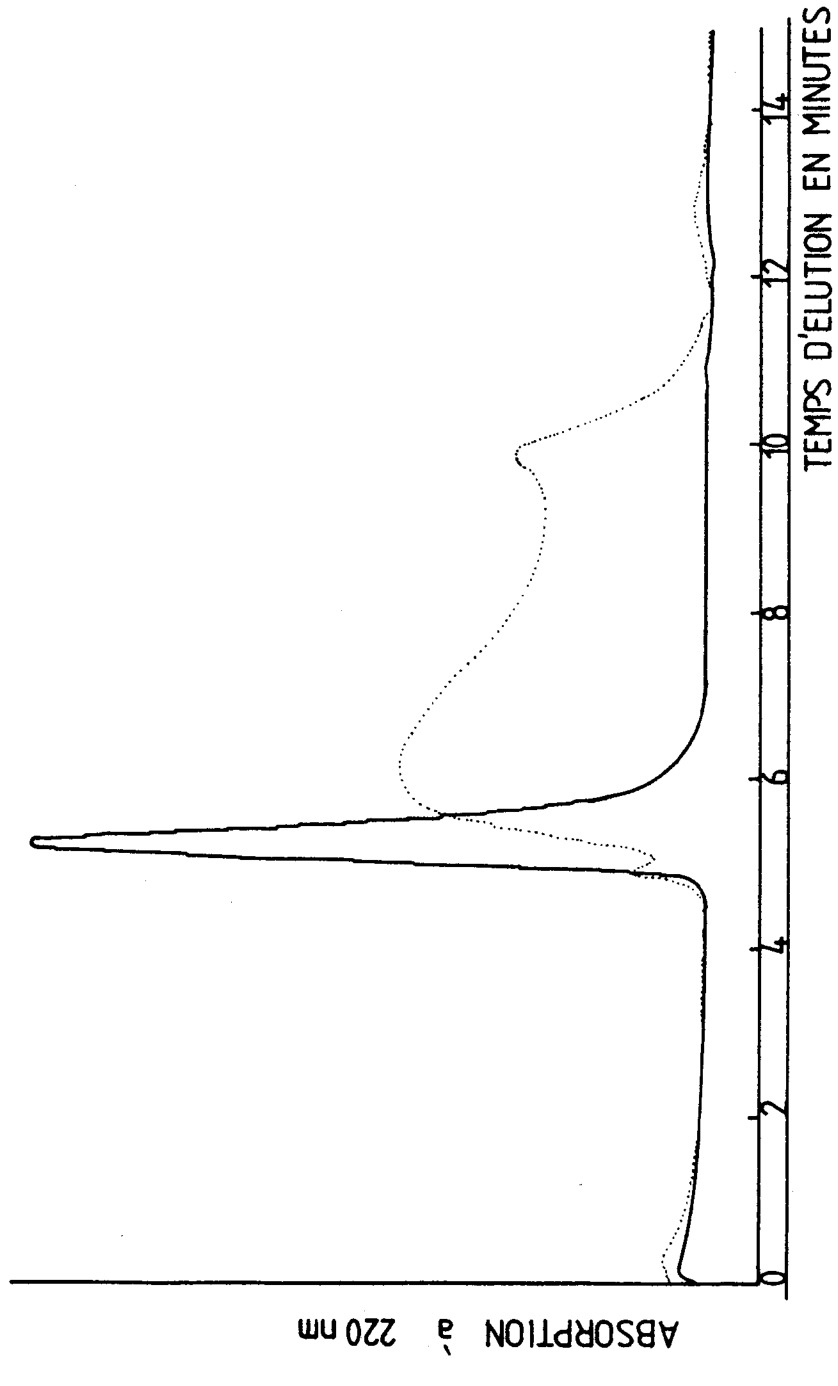


MISE EN EVIDENCE IN-VITRO DE L'ACTIVITE ENDO-β-ELIMINASIQUE

FIG. 3

# Chromatofocusing

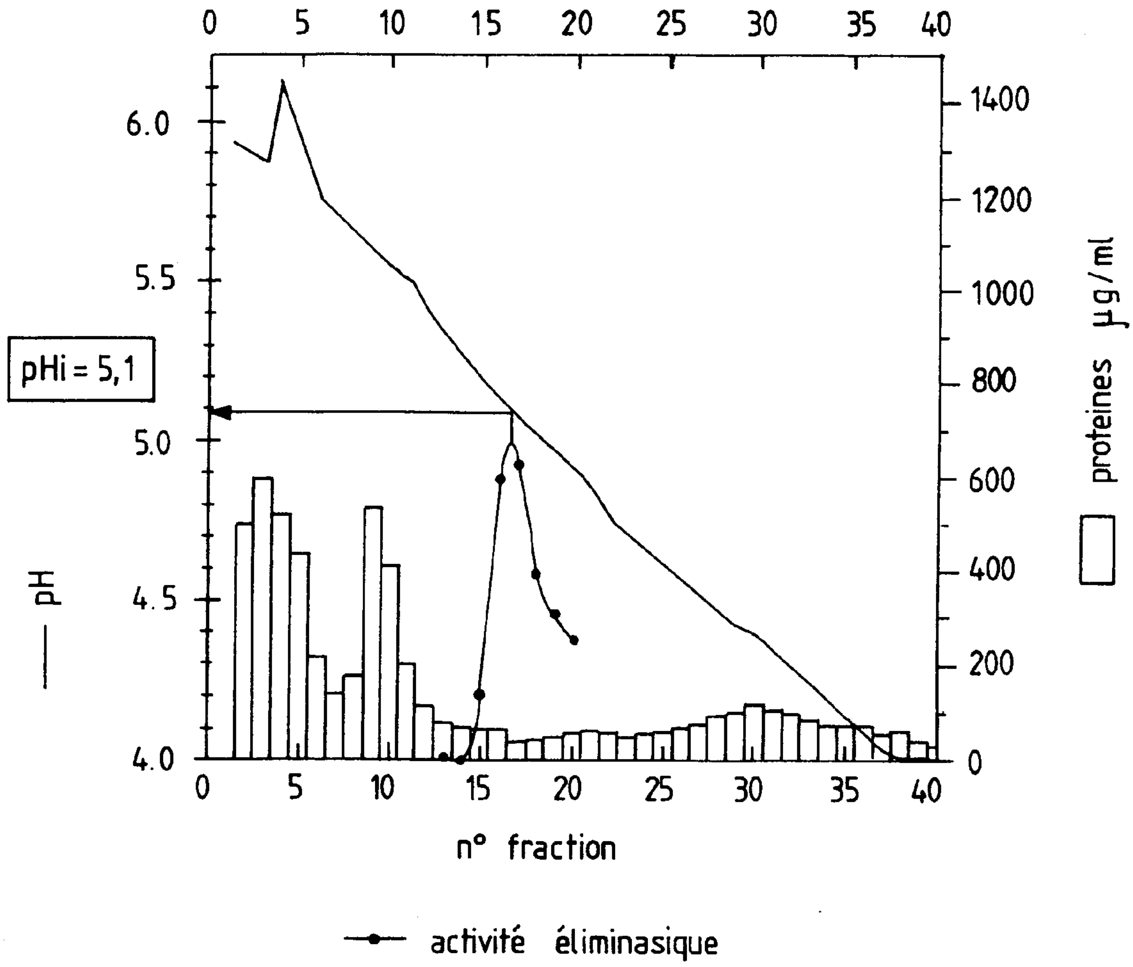


FIG.4

GPC éliminase solubilisée

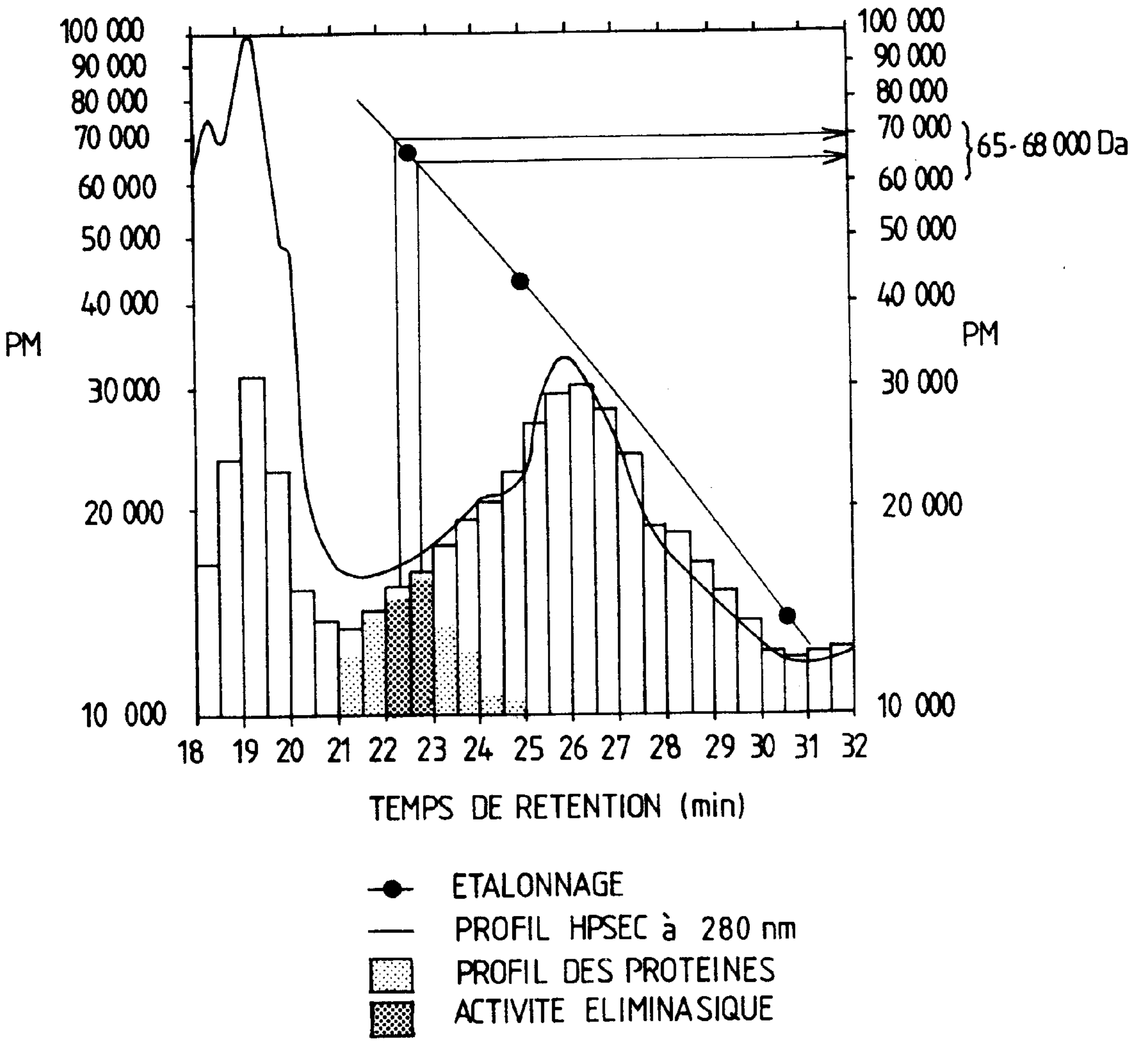


TSK G2000SW 7,5 × 300 mm NaCl 0,3 M 0,3 ml/min

FIG. 5a

GPC Eliminase solubilisée

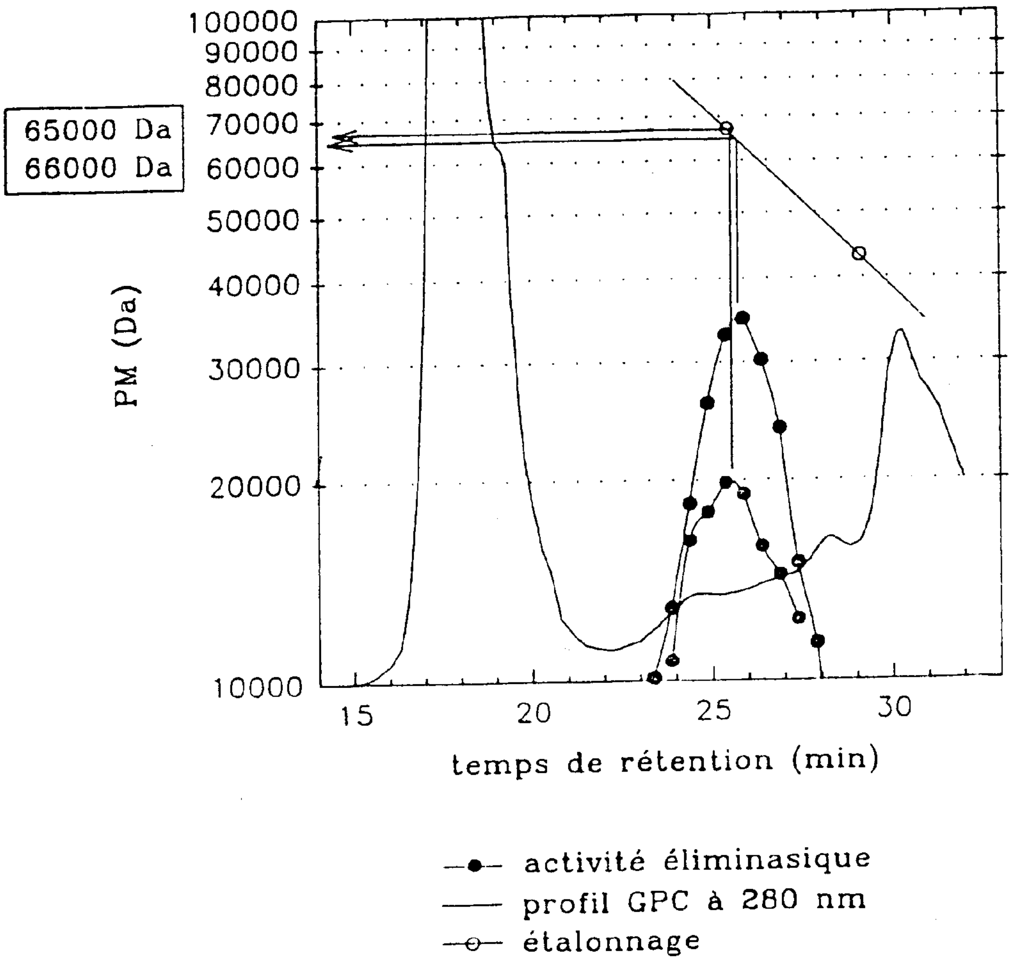


TSK G3000 SW NaCl 0,3 M Tris 50 mM pH 7,00 0,3 ml/min

FIG. 5b

Office européen des brevets

# RAPPORT DE RECHERCHE EUROPEENNE

Numero de la demande
EP 93 40 0478

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,A | EP-A-0 333 243 (AKZO)<br>20 Septembre 1989<br>* revendications; exemples 1,2 *<br>--- | 1 | C12N9/88<br>C12P19/26<br>C08B37/00 |
| A | EUR.J.BIOCHEM<br>vol. 116, 1981,<br>pages 359 - 364<br>W.F.VANN ET AL. 'The structure of the capsular polysaccharide (K5 Antigen) of urinary-tract-infective E. coli 010:K5:H4'<br>* le document en entier *<br>--- | 1 | |
| A | US-A-3 812 012 (L.BUSCHMANN ET AL.)<br>21 Mai 1974<br>* revendications *<br>--- | 1 | |
| A | EP-A-0 294 879 (NEDERLANDSCHE ORGANISATIE VOOR TNO)<br>14 Décembre 1988<br>* revendications *<br>----- | 1 | DOMAINES TECHNIQUES RECHERCHES (Int. Cl.5 )<br>C12N<br>C12P<br>C08B |

Le présent rapport a été établi pour toutes les revendications

| Lieu de la recherche | Date d'achèvement de la recherche | Examinateur |
|---|---|---|
| LA HAYE | 25 MAI 1993 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)